(19) Europäisches Patentamt
European Patent Office
Office européen des brevets

(11) EP 1 231 212 B1

(12) EUROPEAN PATENT SPECIFICATION

(45) Date of publication and mention of the grant of the patent:
**20.12.2006 Bulletin 2006/51**

(51) Int Cl.:
***C07D 471/08*** *(2006.01)* ***C07D 487/08*** *(2006.01)*
***A61K 31/00*** *(2006.01)* ***A61P 25/00*** *(2006.01)*

(21) Application number: **02250540.8**

(22) Date of filing: **28.01.2002**

# (54) Pharmaceutical compositions for the treatment of disorders of the CNS and other disorders

Pharmazeutische Zusammensetzungen zur Behandlung von Störungen des ZNS oder anderen Erkrankungen

Composition pharmaceutique utiles pour le traitement de troubles du SNC ou d'autres troubles

(84) Designated Contracting States:
**AT BE CH CY DE DK ES FI FR GB GR IE IT LI LU MC NL PT SE TR**

(30) Priority: **06.02.2001 US 266749 P**
**12.03.2001 US 274994 P**

(43) Date of publication of application:
**14.08.2002 Bulletin 2002/33**

(73) Proprietor: **Pfizer Products Inc.**
**Groton,**
**Connecticut 06340 (US)**

(72) Inventors:
- **O'Donnell, Christopher John,**
**Pfizer Global**
**Groton,**
**Connecticut 06340 (US)**
- **O'Neill, Brian Thomas,**
**Pfizer Global**
**Groton,**
**Connecticut 06340 (US)**

(74) Representative: **Edwards, Fiona Anne et al**
**Pfizer Limited,**
**UK Patents Department,**
**Ramsgate Road**
**Sandwich,**
**Kent CT13 9NJ (GB)**

(56) References cited:
**WO-A-00/34279**
**WO-A-00/58311**
**US-A- 3 954 766**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Background of the Invention

The present invention relates to CNS-penetrant alpha-7 (α7) nicotinic receptor agonists for the treatment of disorders of the central nervous system (CNS) and other disorders in a mammal, including a human.

It also relates to pharmaceutical compositions containing a pharmaceutically acceptable carrier and a CNS-penetrant alpha-7 nicotinic receptor agonist. WO-A-00/58311 discloses 1,4-diazabicyclo[3.2.2]nonane-4-carboxylate and carboxamide derivatives.

Schizophrenia is characterized by some or all of the following symptoms: delusions (i.e., thoughts of grandeur, persecution, or control by an outside force), auditory hallucinations, incoherence of thought, loss of association between ideas, marked poverty of speech, and loss of emotional responsiveness. Schizophrenia has long been recognized as a complex disease, which to date has eluded biochemical or genetic characterization. However, recent data in the literature suggest that alpha-7 nicotinic receptor agonists may be therapeutic for this, and other CNS disorders, see: Alder, L.E.; Hoffer, L.D.; Wiser, A; Freedman, R. Am. J. Psychiatry 1993, 150, 1856; Bickford, P.C.; Luntz-Leybman, V.; Freedman, R. Brain Research, 1993, 607, 33; Stevens, K.E.; Meltzer, J.; Rose, G.M. Psychopharmacology 1995, 119, 163; Freedman, R.; Coon, H.; Myles-Worsley, M.; Orr-Urtreger, A.; Olincy, A.; Davis, A.; Polymeropoulos, M.; Holik, J.; Hopkins, J.; Hoff, M.; Rosenthal, J.; Waldo, M.C.; Reimherr, F.; Wender, P.; Yaw, J.; Young, D.A.; Breese, C.R.; Adams, C.; Patterson, D.; Alder, L.E.; Kruglyak, L.; Leonard, S.; Byerley, W. Proc. Nat. Acad. Sci. USA 1997, 94, 587.

The compositions of the present invention that contain an alpha-7 nicotinic receptor agonist are useful for the treatment of depression. As used herein, the term "depression" includes depressive disorders, for example, single episodic or recurrent major depressive disorders, and dysthymic disorders, depressive neurosis, and neurotic depression; melancholic depression including anorexia, weight loss, insomnia and early morning waking, and psychomotor retardation; atypical depression (or reactive depression) including increased appetite, hypersomnia, psychomotor agitation or irritability, anxiety and phobias, seasonal affective disorder, or bipolar disorders or manic depression, for example, bipolar disorder, bipolar II disorder and cyclothymic disorder.

Other mood disorders encompassed within the term "depression" include dysthymic disorder with early or late onset and with or without atypical features; dementia of the Alzheimer's type, with early or late onset, with depressed mood; vascular dementia with depressed mood, mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood.

The compositions of the present invention that contain an alpha-7 nicotinic receptor agonist are useful for the treatment of anxiety. As used herein, the term "anxiety" includes anxiety disorders, such as panic disorder with or without agoraphobia, agoraphobia without history of panic disorder, specific phobias, for example, specific animal phobias, social phobias, obsessive-compulsive disorder, stress disorders including post-traumatic stress disorder and acute stress disorder, and generalized anxiety disorders.

"Generalized anxiety" is typically defined as an extended period (e.g. at least six months) of excessive anxiety or worry with symptoms on most days of that period. The anxiety and worry is difficult to control and may be accompanied by restlessness, being easily fatigued, difficulty concentrating, irritability, muscle tension, and disturbed sleep.

"Panic disorder" is defined as the presence of recurrent panic attacks followed by at least one month of persistent concern about having another panic attack. A "panic attack" is a discrete period in which there is a sudden onset of intense apprehension, fearfulness or terror. During a panic attack, the individual may experience a variety of symptoms including palpitations, sweating, trembling, shortness of breath, chest pain, nausea and dizziness. Panic disorder may occur with or without agoraphobia.

"Phobias" includes agoraphobia, specific phobias and social phobias. "Agoraphobia" is characterized by an anxiety about being in places or situations from which escape might be difficult or embarrassing or in which help may not be available in the event of a panic attack. Agoraphobia may occur without history of a panic attack. A "specific phobia" is characterized by clinically significant anxiety provoked by feared object or situation. Specific phobias include the following subtypes: animal type, cued by animals or insects; natural environment type, cued by objects in the natural environment, for example storms, heights or water; blood-injection-injury type, cued by the sight of blood or an injury or by seeing or receiving an injection or other invasive medical procedure; situational type, cued by a specific situation such as public transportation, tunnels, bridges, elevators, flying, driving or enclosed spaces; and other type where fear is cued by other stimuli. Specific phobias may also be referred to as simple phobias. A "social phobia" is characterized by clinically significant anxiety provoked by exposure to certain types of social or performance circumstances. Social phobia may also be referred to as social anxiety disorder.

Other anxiety disorders encompassed within the term "anxiety" include anxiety disorders induced by alcohol, amphetamines, caffeine, cannabis, cocaine, hallucinogens, inhalants, phencychdine, sedatives, hypnotics, anxiolytics and other substances, and adjustment disorders with anxiety or with mixed anxiety and depression.

Anxiety may be present with or without other disorders such as depression in mixed anxiety and depressive disorders. The compositions of the present invention are therefore useful in the treatment of anxiety with or without accompanying depression.

By the use of a CNS-penetrant alpha-7 nicotinic receptor agonist in accordance with the present invention, it is possible to treat depression and/or anxiety in patients for whom conventional antidepressant or antianxiety therapy might not be wholly successful or where dependence upon the antidepressant or antianxiety therapy is prevalent.

Summary of the Invention

This invention relates to compounds of the formula I

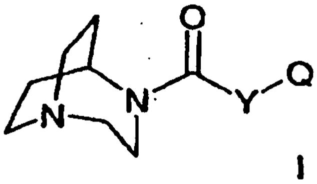

I

wherein

Y = O or NH;

Q is $C_6$- aryl, which is substituted with $R^3$;

$R^3$ is (5-12 membered) heteroaryl that is optionally substituted with from one to five substituents independently selected from H, F, Cl, Br, I, nitro, cyano, $CF_3$, $-NR^6R^7$, $-NR^6C(=O)R^7$, $-NR^6C(=O)NR^7R^8$, $-NR^6S(=O)_2R^7$;$-NR^6S(=O)_2NR^7R^8$, $-OR^8$, $-OC(=O)R^6$, $-OC(=O)OR^6$, $-OC(=O)NR^6R^7$, $-OC(=O)SR^8$, $-C(=O)OR^6$, $-C(=O)R^6$,$-C(=O)NR^6R^7$, $-SR^6$, $-S(=O)R^6$, $-S(=O)_2R^6$, $-S(=O)_2NR^6R^7$, straight chain or branched $(C_1-C_6)$alkyl, straight chain or branched $(C_2-C_6)$alkenyl, straight chain or branched $(C_2-C_8)$alkynyl, $(C_3-C_8)$cycloalkyl, $(C_4-C_8)$cycloalkenyl, (3-8 membered) heterocycloalkyl, $(C_5-C_{11})$bicycloalkyl, $(C_7-C_{11})$bicycloalkenyl, (5-11 membered) heterobicycloalkyl, (5-11 membered) heterobicycloalkenyl, $(C_5-C_{11})$ aryl, (5-12 membered) heteroaryl, and $R^6$;

each $R^6$, $R^7$, and $R^8$ is independently selected from H, straight chain or branched $(C_1-C_8)$alkyl, straight chain or branched $(C_2-C_8)$alkenyl, straight chain or branched $(Cy-C_8)$alkynyl, $(C_3-C_6)$cycloalkyl, $(C_4-C_6)$cycloalkenyl, 3-8 membered heterocycloalkyl, $(C_5-C_{11})$bicycloalkyl, $(C_7-C_{11})$bicycloalkenyl, (5-11 membered)heterobicycloalkyl, (5-11 membered) heterobicycloalkenyl, $(C_6-C_{11})$ aryl and (5-12 membered) heteroaryl; wherein $R^6$, $R^7$, and $R^8$ are each independently optionally substituted with from one to six substituents, independently selected from F, Cl, Br, I, nitro, cyano, $CF_3$, $-NR^9R^{10}$, $-NR^9C(=O)R^{10}$, $-NR^9C(=O)NR^{10}R^{11}$, $-R^9S(=O)_2R^{10}$, $-NR^9S(=O)_2NR^{10}R^{11}$, $-OR^9$, $-OC(=O)R^9$, $-OC(=O)OR^9$, $-OC(=O)NH^9R^{10}$, $-OC(=O)SR^9$, $-C(=O)OR^9$, $-C(=O)R^9$, $-C(=O)NR^6R^7$, $-SR^6$, $-S(=O)R^6$, $-S(=O)_2R^8$, $-S(=O)_2NR^6R^7$, straight chain or branched $(C_1-C_8)$alkyl, straight chain or branched $(C_2-C_8)$alkenyl, straight chain or branched $(C_2-C_8)$alkynyl, $(C_3-C_8)$cycloalkyl, $(C_4-C_8)$cycloalkenyl, 3-8 membered heterocycloalkyl, $(C_5-C_{11})$ bicycloalkyl, $(C_7-C_{11})$bicycloalkenyl, 5-11 membered haterobicycloalkyl, (5-11 membered) heterobicycloalkenyl, $(C_6-C_{11})$ aryl, 5-12 membered heteroaryl, and $R^9$;

each $R^9$, $R^{10}$, and $R^{11}$ is independently selected from H, straight chain or branched $(C_1-C_8)$alkyl, straight chain or branched $(C_2-C_6)$alkenyl, straight chain or branched $(C_2-C_8)$alkynyl, $(C_3-C_8)$cycloalkyl, $(C_4-C_8)$cycloalkenyl, 3-8 membered heterocycloalkyl, $(C_5-C_{11})$bicycloalkyl, $(C_7-C_{11})$bicycloalkenyl, (5-11 membered)heterobicycloalkyl, (5-11 membered) heterobicycloalkenyl, $(C_8-C_{11})$ aryl and (5-12 membered) heteroaryl;

and all enantiomeric, diastereomeric, and tautomeric isomers of such compounds, and pharmaceutically acceptable salts of such compounds and isomers.

More specific embodiments of this invention relate to compounds of the formula I wherein Y = O.

The term "alkyl", as used herein, unless otherwise indicated, includes saturated monovalent hydrocarbon radicals having straight or branched moieties. Examples of alkyl groups include, but are not limited to, methyl, ethyl, propyl, isopropyl, and t-butyl.

The term "alkenyl", as used herein, unless otherwise indicated, includes alkyl moieties having at least one carbon-carbon double bond wherein alkyl is as defined above. Examples of alkenyl include, but are not limited to, ethenyl and propenyl.

The term "alkynyl", as used herein, unless otherwise indicated, includes alkyl moieties having at least one carbon-carbon triple bond wherein alkyl is as defined above. Examples of alkynyl groups include, but are not limited to, ethynyl and 2-propynyl.

The term "cycloalkyl", as used herein, unless otherwise indicated, includes non-aromatic saturated cyclic alkyl moieties wherein alkyl is as defined above. Examples of cycloalkyl include, but are not limited to, cyclopropyl, cyclobutyl, cyclopentyl, cyclohexyl, and cycloheptyl. "Bicycloalkyl" groups are non-aromatic saturated carbocyclic groups consisting of two rings. Examples of bicycloalkyl groups include, but are not limited to, bicyclo-[2.2.2]-octyl and norbornyl. The term "cycloalkenyl" and "bicycloalkenyl" refer to non-aromatic carbocyclic cycloalkyl and bicycloalkyl moieties as defined above, except comprising of one or more carbon-carbon double bonds connecting carbon ring members (an "endocyclic" double bond) and/or one or more carbon-carbon double bonds connecting a carbon ring member and an adjacent non-ring carbon (an "exocyclic" double bond). Examples of cycloalkenyl groups Include, but are not limited to, cyclopentenyl and cyclohexenyl. A non-limiting example of a bicycloalkenyl group is norborenyl. Cycloalkyl, cycloalkenyl, bicycloalkyl, and bicycloalkenyl groups also include groups similar to those described above for each of these respective categories, but which are substituted with one or more oxo moieties. Examples of such groups with oxo moieties include, but are not limited to oxocyclopentyl, oxocyclobutyl, oxocyclopentenyl, and norcamphoryl.

The term "aryl", as used herein, unless otherwise indicated, includes an organic radical derived from an aromatic hydrocarbon by removal of one hydrogen atom. Examples of aryl groups include, but are not limited to phenyl and naphthyl.

The terms "heterocyclic" and "heterocycloalkyl", as used herein, refer to non-aromatic cyclic groups containing one or more heteroatoms, preferably from one to four heteroatoms, each selected from O, S and N. "Heterobicycloalkyl" groups are non-aromatic two-ringed cyclic groups, wherein at least one of the rings contains a heteroatom (O, S, or N). The heterocyclic groups of this invention can also include ring systems substituted with one or more oxo moieties. Examples of non-aromatic heterocyclic groups include, but are not limited to, aziridinyl, azetidinyl, pyrrolidinyl, piperidinyl, azepinyl, piperazinyl, 1,2,3,6-tetrahydropyridinyl, oxiranyl, oxetanyl, tetrahydrofuranyl, tetrahydropyranyl, tetrahydrothienyl, tetrahydrothiopyranyl, piperidino, morpholino, thiomorpholino, thioxanyl, pyrrolinyl, indolinyl, 2H-pyranyl, 4H-pyranyl, dioxanyl, 7,3-dioxolanyl, pyrazolinyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl, quinuclidinyl and quinolizinyl.

The term "heteroaryl", as used herein, refers to aromatic groups containing one or more heteroatoms (O, S, or N). A multicyclic group containing one or more heteroatoms wherein at least one ring of the group is aromatic is a "heteroaryl" group. The heteroaryl groups of this invention can also include ring systems substituted with one or more oxo moieties. Examples of heteroaryl groups include, but are not limited to, pyridinyl, pyridazinyl, imidazolyl, pyrimidinyl, pyrazolyl, triazolyl, pyrazinyl, quinolyl, isoquinolyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, purinyl, oxadiazolyl, thiazolyl, thiadiazolyl, furazanyl, benzofurazanyl, benzothiophenyl, benzotriazolyl, benzothiazolyl, benzoxazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl, and azaindolyl.

The foregoing heteroaryl, heterocyclic and heterocycloalkyl groups may be C-attached or N-attached (where such is possible). For instance, a group derived from pyrrole may be pyrrol-1-yl (N-attached) or pyrrol-3-yl (C-attached).

Examples of specific compounds of this invention are the following compounds of the formula I and their pharmaceutically acceptable salts, hydrates, solvates and optical and other stereoisomers:

1,4-Diaza-bicyclo[3.2.2]nonane-4-carboxylic acid 4-pyridin-2-yl-phenyl ester;
1,4-Diaza-bicyclo[3.2.2]nonane-4-carboxylic acid 4-pyridin-3-yl-phenyl ester;
1,4-Diaza-bicyclo[3.2.2]nonane-4-carboxylic acid 4-pyridin-4-yl-phenyl ester;
1,4-Diaza-bicyclo[3.2.2]nonane-4-carboxylic acid 4-furan-3-yl-phenyl ester;
1,4-Diaza-bicyclo[3.2.2]nonane-4-carboxylic acid 4-(6-fluoro-pyridin-3-yl)-phenyl ester;
1,4-Diaza-bicyclo[3.2.2]nonane-4-carboxylic acid 4-imidazol-1-yl-phenyl ester;
1,4-Diaza-bicyclo[3.2.2]nonane-4-carboxylic acid 4-[1,2,4]triazol-1-yl-phenyl ester;
1,4-Diaza-bicyclo[3.2.2]nonane-4-carboxylic acid 2-benzooxazol-2-yl-phenyl ester;
1,4-Diaza-bicyclo[3.2.2]nonane-4-carboxylic acid 2-benzothiazol-2-yl-phenyl ester;
1,4-Diaza-bicyclo[3.2.2]nonane-4-carboxylic acid 4-(5-ethoxycarbonyl-pyridin-3-yl)-phenyl ester;
1,4-Diaza-bicyclo[3.2.2]nonane-4-carboxylic acid 4-(6-methyl-pyridin-2-yl)-phenyl ester;
1,4-Diaza-bicyclo[3.2.2]nonane-4-carboxylic acid 4-(3,5-dimethyl-isoxazol-4-yl)-phenyl ester;
1,4-Diaza-bicyclo[3.2.2]nonane-4-carboxylic acid 4-(4-methyl-pyridin-2-yl)-phenyl ester;
1,4-Diaza-bicyclo[3.2.2]nonane-4-carboxylic acid 4-(5-carbamoyl-pyridin-3-yl)-phenyl ester;
1,4-Diaza-bicyclo[3.2.2]nonane-4-carboxylic acid 4-(5-cyano-pyridin-3-yl)-phenyl ester;
1,4-Diaza-bicyclo[3.2-2]nonane-4-carboxylic acid 4-imidazo[1,2-a]pyridin-3-yl-phenyl ester; and
1,4-Diaza-bicyclo[3.2.2]nonane-4-carboxylic acid 3-pyridin-3-yl-phenyl ester.

Unless otherwise indicated, the term "one or more substituents", as used herein, refers to from one to the maximum number of substituents possible based on the number of available bonding sites.

The term "treatment", as used herein, refers to reversing, alleviating, inhibiting the progress of, or preventing

the disorder or condition to which such term applies, or one or more symptoms of such condition or disorder. The term "treatment", as used herein, refers to the act of treating, as "treating" is defined immediately above.

Compounds of formula I may contain chiral centers and therefore may exist in different enantiomeric and diastereomeric forms. Individual isomers can be obtained by known methods, such as optical resolution, optically selective reaction, or chromatographic separation in the preparation of the final product or its intermediate. This invention relates to all optical isomers and all stereoisomers of compounds of the formula I, both as racemic mixtures and as individual enantiomers and diastereoismers of such compounds, and mixtures thereof, and to all pharmaceutical compositions and methods of treatment defined above that contain or employ them, respectively.

In so far as the compounds of formula I of this invention are basic compounds, they are all capable of forming a wide variety of different salts with various inorganic and organic acids. Although such salts must be pharmaceutically acceptable for administration to animals, it is often desirable in practice to initially isolate the base compound from the reaction mixture as a pharmaceutically unacceptable salt and then simply convert to the free base compound by treatment with an alkaline reagent and thereafter convert the free base to a pharmaceutically acceptable acid addition salt. The acid addition salts of the base compounds of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained. The acids which are used to prepare the pharmaceutically acceptable acid addition salts of the aforementioned base compounds of this invention are those which form non-toxic acid addition salts, i.e., salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bi-tartrate, succinate, maleate, fumarate, gluconate, saccharate, benzoate, methanesulfonate, ethanesulfonate, benzenesulfonate, p-toluenesulfonate and pamoate (i.e., 1,1'-methylene-bis-(2-hydroxy-3-naphthoate))salts.

The present invention also includes isotopically labelled compounds, which are identical to those recited in formula I, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$, and $^{36}Cl$, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically labelled compounds of the present invention, for example those into which radioactive isotopes such as $^{3}H$ and $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. Further, substitution with heavier isotopes such as deuterium, i.e., $^{2}H$, can afford certain therapeutic advantages resulting from greater metabolic stability, for example increased in vivo half-life or reduced dosage requirements and, hence, may be preferred in some circumstances. Isotopically labelled compounds of formula I of this invention and prodrugs thereof can generally be prepared by carrying out the procedures disclosed In the reaction schemes and/or in the experimental examples below, by substituting a readily available isotopically labelled reagent for a non-isotopically labelled reagent.

The present invention also relates to a pharmaceutical composition for the treatment of schizophrenia in a mammal, including a human, comprising an amount of a compound of the formula I, or a pharmaceutically acceptable salt thereof, that is effective in treating schizophrenia and a pharmaceutically acceptable carrier.

The present invention also relates to the use of a compound of formula I, or a pharmaceutically acceptable salt thereof, in the preparation of a medicament for the treatment of schizophrenia in a mammal, including a human.

The present invention also relates to a pharmaceutical composition for the treatment of schizophrenia in a mammal, including a human, comprising an $\alpha7$ nicotinic receptor agonist compound of the formula I, or a pharmaceutically acceptable salt thereof, and a pharmaceutically acceptable carrier.

The present invention also relates to a pharmaceutical composition for treating a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotropic lateral sclerosis (ALS), cognitive dysfunction, tinnitus, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbiturates, opioids or cocaine), headache, stroke, traumatic brain injury (TBI), psychosis, Huntington's Chorea, tardive dyskinesia, hyperkinesia; dyslexia, mulfi-infarct dementia, age related cognitive decline, epilepsy, including petit mal absence epilepsy, HIV induced dementia, senile dementia of the Alzheimer's type (AD), Parkinson's disease (PD), attention deficit hyperactivity disorder (ADHD) and Tourette's Syndrome in a mammal, comprising an amount of a compound of the formula I, or a pharmaceutically acceptable salt thereof, that is effective in treating such disorder or condition and a pharmaceutically acceptable carrier.

The present invention also relates to the use of a compound of formula I, or a pharmaceutically acceptable salt thereof, in the preparation of a medicament for the treatment of a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotropic lateral sclerosis (ALS), cognitive dysfunction, tinnitus, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke, traumatic brain injury (TBI), psychosis, Huntington's Chorea, tardive dyskinesia, hyperkinesia, dyslexia, multi-infarct dementia, age related cognitive decline, epilepsy, including petit mal absence epilepsy, HIV induced dementia, senile dementia of the Alzheimer's type (AD), Parkinson's disease (PD), attention deficit hyperactivity disorder (ADHD) and Tourette's Syndrome in a mammal.

The present invention also relates to a pharmaceutical composition for treating a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotropic lateral sclerosis (ALS), cognitive dysfunction, tinnitus, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke, traumatic brain injury (TBI), psychosis, Huntington's Chorea, tardive dyskinesia, hyperkinesia, dyslexia, multi-infarct dementia, age related cognitive decline, epilepsy, including petit mal absence epilepsy, HIV induced dementia, senile dementia of the Alzheimer's type (AD), Parkinson's disease (PD), attention deficit hyperactivity disorder (ADHD) and Tourette's Syndrome in a mammal, comprising an a7 nicotinic receptor agonizing amount of a compound of the formula I, or a pharmaceutically acceptable salt thereof, and a pharmaceutically acceptable carrier.

The present invention also relates to the use of a compound of formula I, or a pharmaceutically acceptable salt thereof, in the preparation of a medicament for the treatment of a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotropic lateral sclerosis (ALS), cognitive dysfunction, tinnitus, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke, traumatic brain injury (TBI), psychosis, Huntington's Chorea, tardive dyskinesia, hyperkinesia, dyslexia, mufti-infarct dementia, age related cognitive decline, epilepsy, including petit mal absence epilepsy, HIV induced dementia, senile dementia of the Alzheimer's type (AD), Parkinson's disease (PD), attention deficit hyperactivity disorder (ADHD) and Tourette's Syndrome in a mammal.

## DETAILED DESCRIPTION OF THE INVENTION

Compounds of the formula I can be readily prepared according to the methods described below. In the reaction schemes and discussion that follow, Y, unless otherwise indicated, is defined as it is above in the definition of compounds of the formula I.

As used herein, the expression "reaction inert solvent" refers to a solvent system in which the components do not interact with starting materials, reagents, or intermediates of products in a manner which adversely affects the yield of the desired product.

During any of the following synthetic sequences discussed below it may be necessary and/or desirable to protect sensitive or reactive groups on any of the molecules concerned. This may be achieved by means of conventional protecting groups, such as those described in T. W. Greene and P. G. M. Wuts, Protective Groups in Organic Synthesis, John Wiley & Sons, 1999.

## Scheme 1

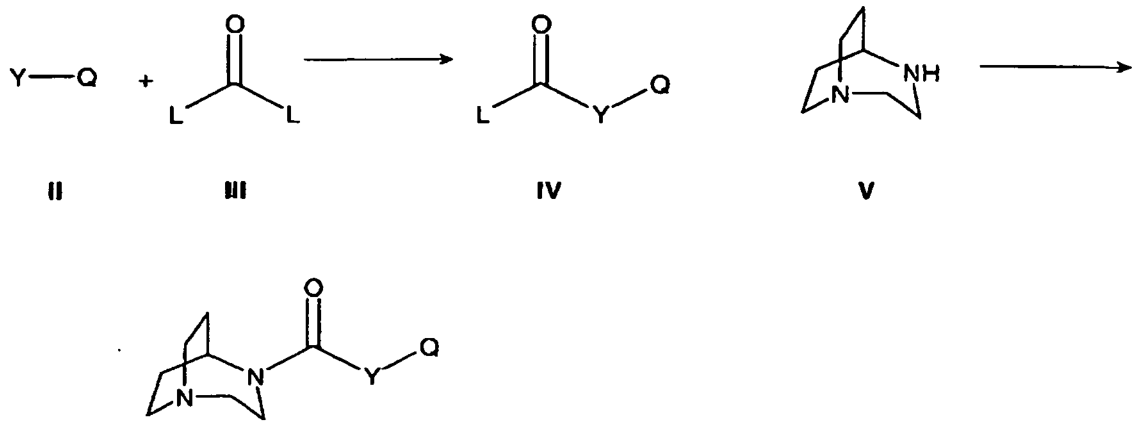

II III IV V

I

Compounds of the formula I may be prepared as outlined in Scheme 1. Referring to Scheme I, a compound of the formula II is reacted with a carbonyl donating compound of the formula III, wherein L is a leaving group, for example, chloride, bromide, imidazole, triazole, tetrazole, trichloromethoxy, thiophenol, phenol or substituted phenol (e.g., p-nitrophenol, p-bromophenol, trichloro or trifluoromethyl), preferably chloride, in the presence of a base, for example, triethylamine, diisopropylamine, pyridine, 2,6-lutidine, sodium or potassium hydroxide, sodium or potassium carbonate or bicarbonate, diisopropylethylamine or 1,8-diazabicyclo[5.4.0]undec-7-ene, preferably triethylamine. This reaction is typically carried out in a reaction inert solvent such as water, acetonitrile, methylene chloride, chloroform, 1,2-dichloroethane, tetrahydrofuran, diethylether, dioxane, 1,2-dimethoxyethane, benzene, or toluene, preferably toluene, at a temperature from about -50°C to about 110°C, preferably from about 0°C to about 50°C. Upon consumation of the compound of formula II, the resulting compound of formula IV is reacted immediately with additional base such as triethylamine, diisopropylamine, pyridine, 2,6-lutidine or 1,8-diazabicyclo[5.4.0]undec-7-ene or any of the other bases referred to above, preferably triethylamine, in the presence or absence of 4-dimethylaminopyridine or polymer supported 4-dimethylaminopyridine, and with a compound of the formula V at a temperature of from about -10°C to about 110°C, preferably from about 25°C to about 110°C, affording the desired compound of formula I.

Alternatively, commercially available compounds of formula IV can be reacted with a compound of formula V in the presence of a base such as triethylamine, diisopropylamine, pyridine, 2,6-lutidine or 1,8-diazabicyclo[5.4.0]undec-7-ene or any of the other bases discussed above, with triethylamine being preferred, in the presence or absence of 4-dimethylaminopyridine or polymer supported 4-dimethylaminopyridine, at a temperature from about -10°C to about 110°C, with from about -10°C to about 25°C being preferred, affording the desired compound of formula I.

Scheme 2

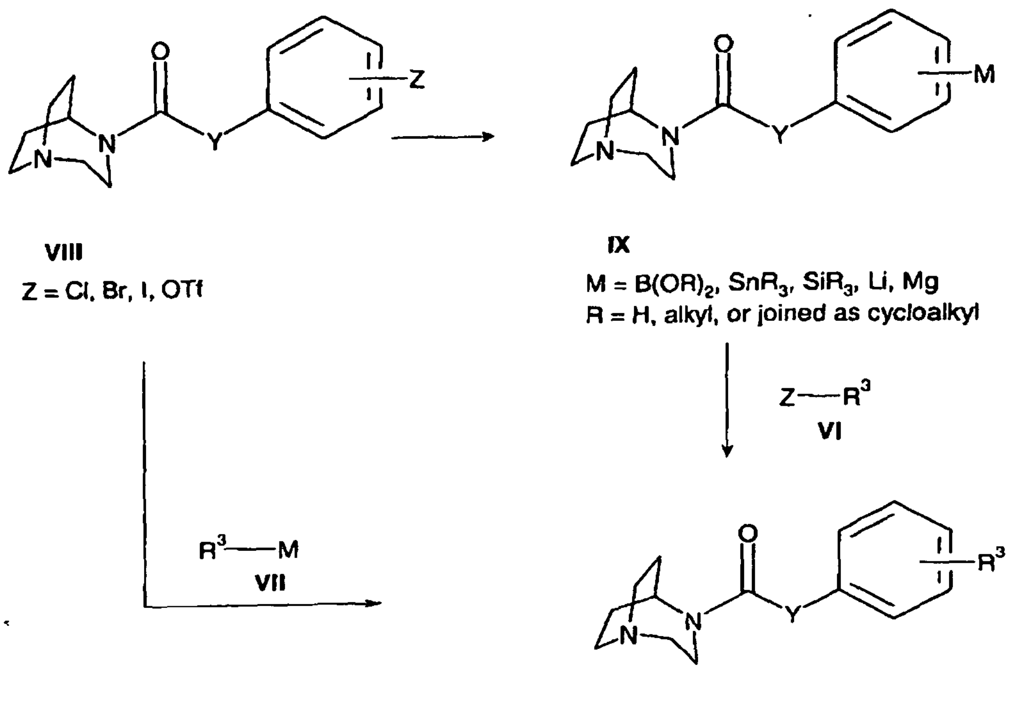


VIII
Z = Cl, Br, I, OTf

IX
M = $B(OR)_2$, $SnR_3$, $SiR_3$, Li, Mg
R = H, alkyl, or joined as cycloalkyl

IA

Scheme 2 illustrates the preparation of compounds of the formula I. Referring to Scheme 2, treatment of a compound of the formula VIII wherein Z is chloro, bromo, iodo or triflate (OTf) with bis(pinacolato)diboron and a palladium catalyst such as palladium (0) tetrakis(triphenylphosphine), palladium (II) acetate, allyl palladium chloride dimer, tris (dibenzylideneacetone)dipalladium (0), tris(dibenzylidene-acetone)dipalladium (0) chloroform adduct, palladium (II) chloride or dichloro[1,1'-bis(diphenylphosphino)ferrocene]palladium (II) dichloromethane adduct, preferably dichloro[1,1'-bis (diphenylphosphino)-ferrocene]palladium (II) dichloromethane adduct, in the presence or absence of a phosphine ligand such as 1,1'-bis(diphenylphosphino)ferrocene, triphenylphosphine, tri-o-tolylphosphine, tri-tert-butylphosphine, 1,2-bis (diphenylphosphino)ethane, 1,3-bis(diphenylphosphino)-propane, BINAP, 2-biphenyl dicyclohexylphosphine, 2-biphenyl-di-tert-butylphosphine, 2-(N,N-dimethylamino)-2'-di-tert-butylphosphino-biphenyl or 2-(N,N-dimethylamino)-2'-dicyclohexylphosphinobiphenyl, preferably 1,1'-bis(diphenylphosphino)ferrocene, and in the presence or absence of a base such as potassium acetate, sodium acetate, cesium acetate, sodium carbonate, lithium carbonate, potassium carbonate, cesium carbonate or cesium fluoride, preferably potassium acetate, yields a compound of the formula IX wherein the Z group has been replaced with M, wherein M = borane pinacol ester. Generally, this reaction is carried out in a reaction inert solvent such as 1,4-dioxane, acetonitrile, methyl sulfoxide, tetrahydrofuran, ethanol, methanol, 2-propanol, toluene, preferably methyl sulfoxide, at a temperature from about from 0°C to about 200°C, preferably from about 80°C to about 120°C.

Other methods of converting a compound of the formula VIII with the Z group mentioned above into a compound of the formula IX wherein the Z group is replaced with M, wherein M is boronic acid, boronic acid ester or trialkylstannane, are known in the art. For instance, treatment of a compound of the formula VIII, wherein Z is Br or I, with an alkyl lithium reagent such as, but not limited to n-butyl lithium, sec butyl lithium or tert-butyl lithium, in a solvent such as diethyl ether, tetrahydrofuran, dimethoxyethane, hexane, toluene, dioxane or a similar reaction inert solvent, at a temperature from about -100°C to about 25°C affords the corresponding compound of the formula IX wherein Z is Li. Treatment of a solution of this material with a suitable boronic ester such as trimethoxyborane, triethoxyborane or triisopropylborane, followed by a standard aqueous work-up with acid will afford the corresponding compound of the formula IX wherein M is boronic acid.

Alternatively, treating a mixture of a compound of the formula VIII wherein Z is Br or I and a boronic ester with an alkyl lithium reagent, as described above, followed by a standard aqueous work-up with acid will afford the corresponding compound of formula IX wherein M is boronic acid. Alternatively, treating a compound of the formula VIII wherein Z is Br or I with an alkyl lithium reagent such as, but not limited to n-butyl lithium, sec butyl lithium or tert-butyl lithium, in a solvent such as diethyl ether, tetrahydrofuran, dimethoxyethane, hexane, toluene, dioxane or a similar

reaction inert solvent, at a temperature from about -100°C to about 25°C will afford the corresponding compound of the formula IX wherein M is Li. Treatment of a solution of this material with a suitable trialkylstannyl halide such as, but not limited to trimethylstannyl chloride or bromide or tributylstannyl chloride or bromide, followed by a standard aqueous work-up will afford the corresponding compound of the formula IX wherein M is trimethyl or tributylstannane.

Treatment of a compound of the formula IX wherein M is a boronic acid, boronic ester, or trialkylstannane group, with an aryl or heteroaryl chloride, aryl or heteroaryl bromide, aryl or heteroaryl iodide, or aryl or heteroaryl triflate of the formula VI, preferably an aryl or heteroaryl bromide, with a palladium catalyst such as palladium (0) tetrakis (triphonylphosphine), palladium (II) acetate, allyl palladium chloride dimer, tris(dibenzylideneacetone)dipalladium (0), tris(dibenzylideneacetone)dipalladium (0) chloroform adduct, palladium (II) chloride or dichloro[1,1'-bis(diphenylphosphino)ferrocene]palladium (II) dichloromethane adduct, preferably dichloro[1,1'-bis(diphenylphosphino)-ferrocene]palladium (II) dichloromethane adduct, in the presence or absence of a phosphine ligand such as 1,1'-bis(diphenylphosphino) ferrocene, triphenylphosphine, tri-o-tolylphosphine, tri-tert-butylphosphine, 1,2-bis(diphenylphosphino)ethane, 1,3-bis (diphenylphosphino)-propane, BINAP, 2-biphenyl dicyclohexylphosphine, 2-biphenyl-di-tert-butylphosphine, 2-(N,N-dimethylamino)-2'-di-tert-butylphosphino-biphenyl or 2-(N,N-dimethylamino)-2'-dicyclohexylphosphinobiphenyl, preferably 1,1'-bis(diphenylphosphino)ferrocene, and in the presence or absence of a base such as potassium phosphate, potassium acetate, sodium acetate, cesium acetate, sodium carbonate, lithium carbonate, potassium carbonate, cesium fluoride or cesium carbonate, preferably potassium phosphate, affords a compound of formula IA. This reaction is typically carried out in a reaction inert solvent such as 1,4-dioxane, acetonitrile, methyl sulfoxide, tetrahydrofuran, ethanol, methanol, 2-propanol, or toluene, preferably 1,4-dioxane, in the presence or absence of from about 1%-about 10% water, preferably about 5% water, at a temperature from about 0°C to about 200°C, preferably from about 60°C to about 100°C.

Alternatively, a compound of the formula VIII can be reacted with a compound of the formula VII, wherein M is a boronic acid, boronic acid ester, borane pinacol ester or trialkylstannane group, using similar reaction conditions as described above, to yield the corresponding compound of formula IA.

Scheme 3

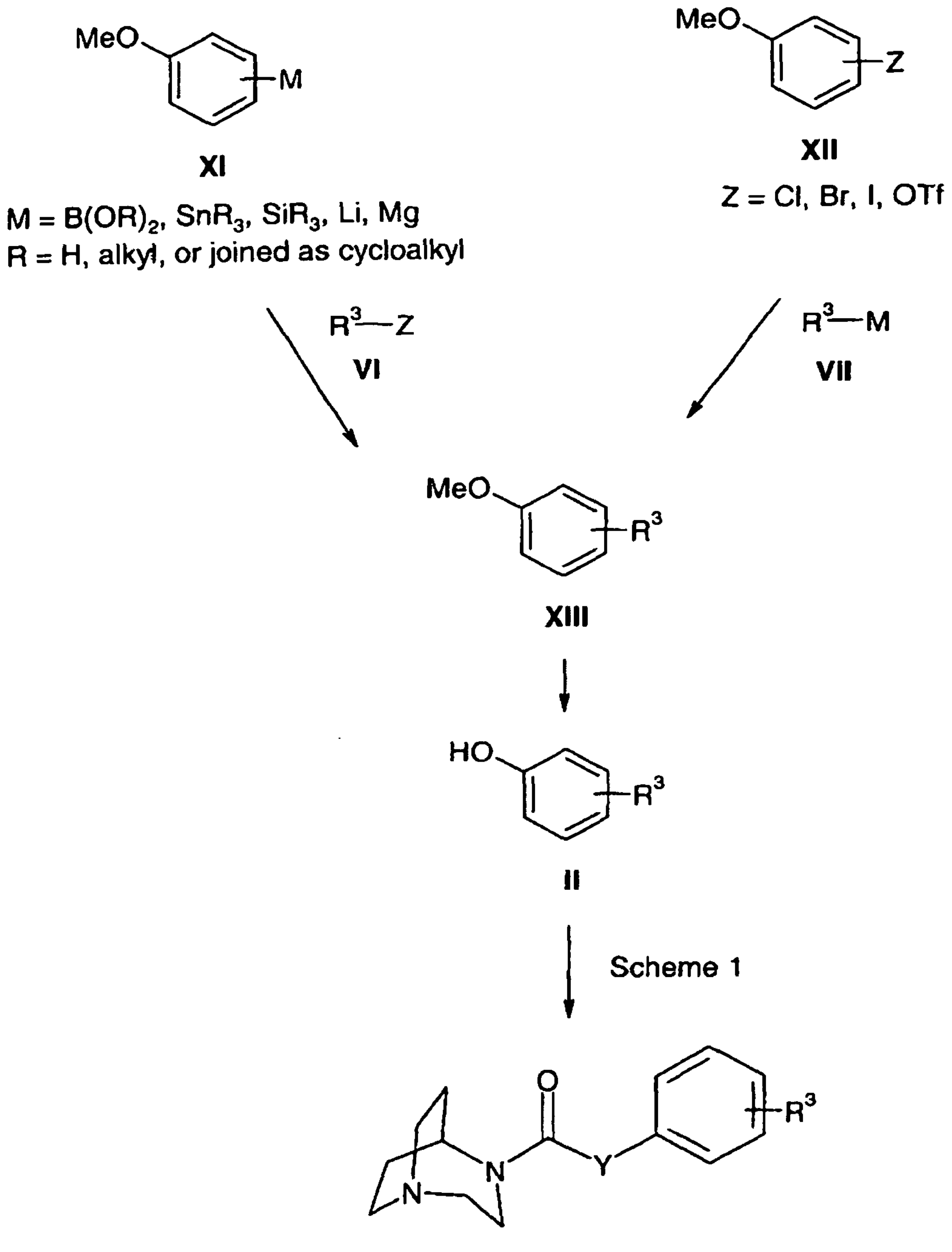


XI

M = $B(OR)_2$, $SnR_3$, $SiR_3$, Li, Mg
R = H, alkyl, or joined as cycloalkyl

XII

Z = Cl, Br, I, OTf

XIII

II

IA

Scheme 3 illustrates an alternative method of preparing compounds of the formula I. Referring to Scheme 3, treatment of a methoxy aryl or heteroaryl ring compound of the formula XI, wherein M = boronic acid, boronic acid ester or a trialkylstannane group, preferably a boronic acid group, with an aryl or heteroaryl chloride, aryl or heteroaryl bromide, aryl or heteroaryl iodide, or aryl or heteroaryl alkoxytriflate of the formula VI wherein Z is defined as above, preferably an aryl or heteroaryl bromide, and with a palladium catalyst such as palladium (0) tetrakis(triphenylphosphine), palladium (II) acetate, allyl palladium chloride dimer, tris(dibenzylideneacetone)dipalladium (0), tris(dibenzylideneacetone)dipalladium (0) chloroform adduct, palladium (II) chloride or dichloro[1,1'-bis(diphenylphosphino)ferrocene]palladium (II) dichloromethane adduct, preferably palladium (0) tetrakis(triphenylphosphine), in the presence or absence of a phosphine ligand such as 1,1'-bis(diphenylphosphino)ferrocene, triphenylphosphine, tri-o-tolylphosphine, tri-tert-butylphosphine, 1,2-bis(diphenylphosphino)ethane, 1,3-bis(diphenylphosphino)- propane, BINAP, 2-biphenyl dicyclohexylphosphine, 2-biphenyl-di-tert-butylphosphine, 2-(N,N-dimethylamino)-2'-di-tert-butylphosphinobiphenyl or 2-(N,N-dimethylamino)-2'-dicyclohexylphosphinobiphenyl, and in the presence or absence of a base such as potassium phosphate, potassium acetate, sodium acetate, cesium acetate, sodium carbonate, lithium carbonate, potassium carbonate, cesium fluoride or cesium carbonate, preferably sodium carbonate, affords a compound of the formula XIII. Examples of suitable reaction inert solvents for this reaction are 1,4-dioxane, acetonitrile, methyl sulfoxide, tetrahydrofuran, ethanol, methanol, 2-propanol and toluene, with ethanol being preferred. This reaction can be carried out in the presence or absence of from about 1% to about 14% water, with about 5% water being preferred. The reaction temperature can range from about 0°C to about 200°C, and is preferably from about 60°C to about 100°C.

An alternative method for the preparation of compounds of the formula XIII from a methoxy aryl or heteroaryl ring substituted with a chloride, bromide, iodide or alkoxytriflate group (*i.e.*, a compound of the formula XII) and an aryl or heteroaryl boronic acid, boronic acid ester, or a trialkylstannane group (*i.e.,* a compound of the formula VII) can be performed using a similar procedure to the one described above.

The methoxy group of the compound of formula XIII can be removed, as described in T. W. Greene and P. G. M. Wuts, Protective Groups in Organic Synthesis, John Wiley & Sons, 1999, p250-254, to generate a compound of formula II. The reaction is preferably carried out using hydrobromic acid at a temperature from about room temperature to about 150°C, preferably from about 80°C to about 110°C.

Scheme 4

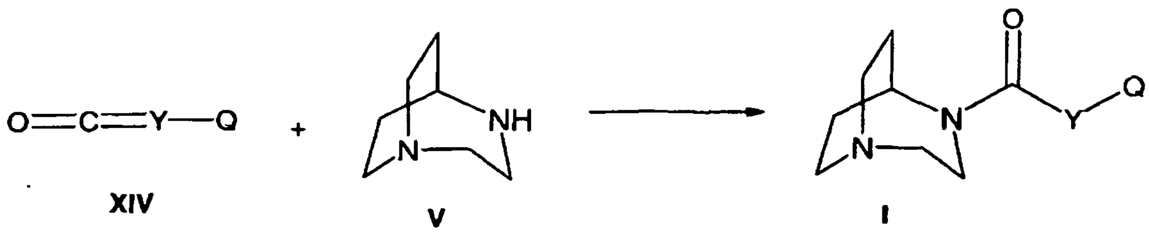

XIV V I

Scheme 4 illustrates the synthesis of compounds of the formula I wherein Y is NH. Referring to Scheme 4, treatment of a compound of formula V with a compound of formula XIV wherein Y is nitrogen in a reaction inert solvent such as acetonitrile, benzene, chloroform, dichloromethane, diethyl ether, dimethylformamide, methyl sulfoxide, ethyl acetate, tetrahydrofuran, or toluene, preferably tetrahydrofuran, at a temperature from about -50°C to about 100°C, preferably from about 0°C to about 50°C, will provide the corresponding compound of formula I where Y is NH.

Scheme 5

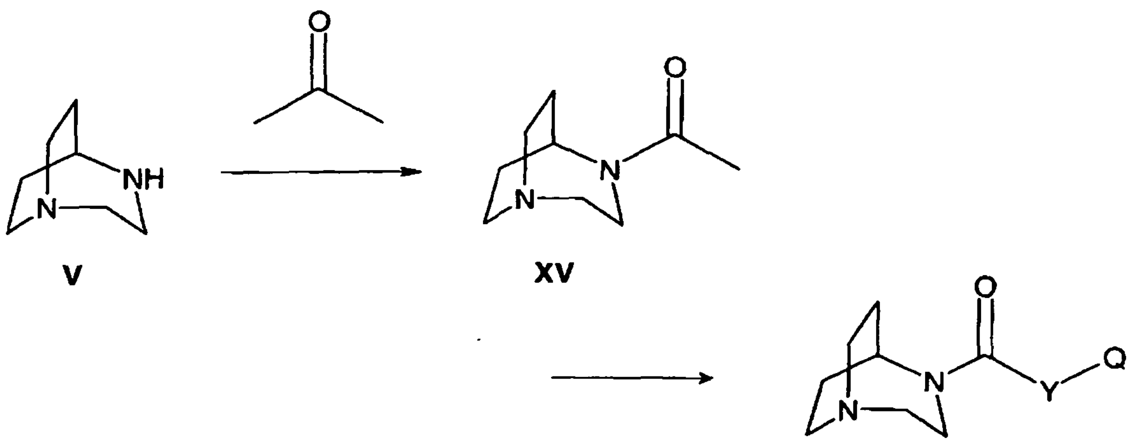

V XV

I

Scheme 5 illustrates a method of preparing compounds of the formula I wherein Y is O or NH. Referring to Scheme 5, treatment of a compound of the formula V with a compound of the formula III, wherein L is a leaving group, for example, chloride, bromide, imidazole, triazole, tetrazole, trichloromethoxy, thiophenol, phenol or substituted phenol (e.g., p-nitrophenol, p-bromophenol, trichloro or trifluoromethyl), preferably chloride, in the presence of a base, for

example, triethylamine, diisopropylamine, pyridine, 2,6-lutidine, sodium or potassium hydroxide, sodium or potassium carbonate or bicarbonate, diisopropylethylamine or 1,8-diazabicyclo[5.4.0]undec-7-ene, preferably triethylamine. This reaction is typically carried out in a reaction inert solvent such as water, acetonitrile, methylene chloride, chloroform, 1,2-dichloroethane, tetrahydrofuran, diethylether, dioxane, 1,2-dimethoxyethane, benzene, or toluene, preferably toluene, at a temperature from about -50°C to about 110°C, preferably from about 0°C to about 50°C and affords the corresponding compound of formula XV. Treatment of the resulting compound of formula XV with a suitable phenol or substituted phenol or an aniline or substituted aniline in the presence or absence of a base such as triethylamine, diisopropylamine, pyridine, 2,6-lutidine or 1,8-diazabicyclo[5.4.0]undec-7-ene or any of the other bases discussed above, with triethylamine being preferred, in a reaction inert solvent such as water, acetonitrile, methylene chloride, chloroform, 1,2-dichloroethane, tetrahydrofuran, diethylether, dioxane, 1,2-dimethoxyethane, benzene, or toluene, preferably toluene at a temperature from about -10°C to about 110°C, with from about -10°C to about 50°C being preferred, affords the desired compound of formula I.

Isolation and purification of the products is accomplished by standard procedures that are known to a chemist of ordinary skill.

In each of the reactions discussed above, or illustrated in Schemes 1-5 above, pressure is not critical unless otherwise indicated. Pressures from about 0.5 atmospheres to about 5 atmospheres are generally acceptable, with ambient pressure, i.e., about 1 atmosphere, being preferred as a matter of convenience.

The compounds of the formula I and their pharmaceutically acceptable salts (hereafter "the active compounds") can be administered via either the oral, transdermal (e.g., through the use of a patch), intranasal, sublingual, rectal, parenteral or topical routes. Transdermal and oral administration are preferred. These compounds are, most desirably, administered in dosages ranging from about 0.25 mg up to about 1500 mg per day, preferably from about 0.25 to about 300 mg per day in single or divided doses, although variations will necessarily occur depending upon the weight and condition of the subject being treated and the particular route of administration chosen. However, a dosage level that is in the range of about 0.01 mg to about 10 mg per kg of body weight per day is most desirably employed. Variations may nevertheless occur depending upon the weight and condition of the persons being treated and their individual responses to said medicament, as well as on the type of pharmaceutical formulation chosen and the time period and interval during which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds can be administered alone or in combination with pharmaceutically acceptable carriers or diluents by any of the several routes previously indicated. More particularly, the active compounds can be administered in a wide variety of different dosage forms, e.g., they may be combined with various pharmaceutically acceptable inert carriers in the form of tablets, capsules, transdermal patches, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents. In addition, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc can be used for tabletting purposes. Solid compositions of a similar type may also be employed as fillers in gelatin capsules; preferred materials in this connection also include lactose or milk sugar, as well as high molecular weight polyethylene glycols. When aqueous suspensions and/or elixirs are desired for oral administration the active ingredient may be combined with various sweetening or flavoring agents, coloring matter and, if so desired, emulsifying and/or suspending agents, together with such diluents as water, ethanol, propylene glycol, glycerin and various combinations thereof.

For parenteral administration, a solution of an active compound in either sesame or peanut oil or in aqueous propylene glycol can be employed. The aqueous solutions should be suitably buffered (preferably pH greater than 8), if necessary, and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

It is also possible to administer the active compounds topically and this can be done by way of creams, a patch, jellies, gels, pastes, ointments and the like, in accordance with standard pharmaceutical practice.

The effectiveness of the active compounds in suppressing nicotine binding to specific receptor sites can be determined by the following procedure, which is a modification of the methods of Lippiello, P. M. and Fernandes, K. G. (in "The Binding of L-[$^3$H]Nicotine To A Single Class of High-Affinity Sites in Rat Brain Membranes", Molecular Pharm.,

29, 448-54, (1986)) and Anderson, D. J. and Arneric, S. P. (in "Nicotinic Receptor Binding of $^3$H-Cystisine, $^3$H-Nicotine and $^3$H-Methylcarmbamylcholine In Rat Brain", European J. Pharm., 253, 261-67 (1994)). Male Sprague-Dawley rats (200-300 g) from Charles River were housed in groups in hanging stainless steel wire cages and were maintained on a 12 hour light/dark cycle (7 a.m.-7 p.m. light period). They received standard Purina Rat Chow and water ad libitum. The rats were killed by decapitation. Brains were removed immediately following decapitation. Membranes were prepared from brain tissue according to the methods of Lippiello and Fernandez (Molec. Pharmacol., 29, 448-454, (1986)) with some modifications. Whole brains were removed, rinsed with ice-cold buffer, and homogenized at 0°C in 10 volumes of buffer (w/v) using a Brinkmann Polytron™ (Brinkmann Instruments Inc., Westbury, NY), setting 6, for 30 seconds. The buffer consisted of 50 mM Tris HCl at a pH of 7.5 at room temperature. The homogenate was sedimented by centrifugation (10 minutes; 50,000 x g; 0° to 4°C). The supernatant was poured off and the membranes were gently resuspended with the Polytron and centrifuged again (10 minutes; 50,000 x g; 0°C to 4°C). After the second centrifugation, the membranes were resuspended in assay buffer at a concentration of 1.0g/100mL. The composition of the standard assay buffer was 50 mM Tris HCl, 120 mM NaCl, 5 mM KCl, 2 mM $MgCl_2$, 2 mM $CaCl_2$ and had a pH of 7.4 at room temperature.

Routine assays were performed in borosilicate glass test tubes. The assay mixture typically consisted of 0.9 mg of membrane protein in a final incubation volume of 1.0 mL. Three sets of tubes were prepared wherein the tubes in each set contained 50$\mu$L of vehicle, blank, or test compound solution, respectively. To each tube was added 200$\mu$L of [$^3$H]-nicotine in assay buffer followed by 750$\mu$L of the membrane suspension. The final concentration of nicotine in each tube was 0.9 nM. The final concentration of cytisine in the blank was 1$\mu$M. The vehicle consisted of deionized water containing 30$\mu$L of 1 N acetic acid per 50 mL of water. The test compounds and cytisine were dissolved in vehicle. Assays were initiated by vortexing after addition of the membrane suspension to the tube. The samples were incubated at 0°C to 4°C in an iced shaking water bath. Incubations were terminated by rapid filtration under vacuum through Whatman GF/B™ glass fiber filters (Brandel Biomedical Research & Development Laboratories, Inc., Gaithersburg, MD) using a Brandel™ multi-manifold tissue harvester (Brandel Biomedical Research & Development Laboratories, Inc., Gaithersburg, MD). Following the initial filtration of the assay mixture, filters were washed two times with ice-cold assay buffer (5 ml each). The filters were then placed in counting vials and mixed vigorously with 20 ml of Ready Safe™ (Beckman, Fullerton, CA) before quantification of radioactivity. Samples were counted in a LKB Wallac Rackbeta™ liquid scintillation counter (Wallac Inc., Gaithersburg, MD) at 40-50% efficiency. All determinations were in triplicate.

Calculations: Specific binding (C) to the membrane is the difference between total binding in the samples containing vehicle only and membrane (A) and non-specific binding in the samples containing the membrane and cytisine (B), i.e.,

$$\text{Specific binding} = (C) = (A) - (B).$$

Specific binding in the presence of the test compound (E) is the difference between the total binding in the presence of the test compound (D) and non-specific binding (B), i.e., (E) = (D) - (B).

$$\%\ \text{Inhibition} = (1-((E)/(C)) \text{ times } 100.$$

The compounds of the invention that were tested in the above assay exhibited $IC_{50}$ values of less than 10$\mu$M.

[$^{125}$I]-Bungarotoxin binding to nicotinic receptors in $GH_4Cl$ cells: Membrane preparations were made for nicotinic receptors expressed in $GH_4Cl$ cell line. Briefly, one gram of cells by wet weight were homogenized with a polytron in 25 mls of buffer containing 20 mM Hepes, 118 mM NaCl, 4.5 mM KCl, 2.5 mM $CaCl_2$, 1.2 mM $MgSO_4$, pH 7.5. The homogenate was centrifuged at 40,000 x g for 10 min at 4°C, the resulting pellet was homogenized and centrifuged again as described above. The final pellet was resuspended in 20 mls of the same buffer. Radioligand binding was carried out with [$^{125}$I] alpha-bungarotoxin from New England Nuclear, specific activity about 16 $\mu$Ci/ ug, used at 0.4 nM final concentration in a 96 well microtiter plate. The plates were incubated at 37°C for 2 hours with 25 $\mu$l drugs or vehicle for total binding, 100 ul [$^{125}$I] Bungarotoxin and 125 ul tissue preparation. Nonspecific binding was determined in the presence of methyllycaconitine at 1 uM final concentration. The reaction was terminated by filtration using 0.5% Polyethylene imine treated Whatman GF/B™ glass fiberfilters (Brandel Biomedical Research & Development Laboratories, Inc., Gaithersburg, MD) on a Skatron cell harvester (Molecular Devices Corporation, Sunnyvale, CA) with ice-cold buffer, filters were dried overnight, and counted on a Beta plate counter using Betaplate Scint. (Wallac Inc., Gaithersburg, MD). Data are expressed as IC50's (concentration that inhibits 50% of the specific binding) or as an apparent Ki, IC50/1 +[L] /KD. [L] = ligand concentration, KD = affinity constant for [$^{125}$I] ligand determined in separate experiment.

[$^{125}$I]-Bungarotoxin binding to alpha1 nicotinic receptors in Torpedo electroplax membranes: Frozen Torpedo

electroplax membranes (100 μl) were resuspended in 213 mls of buffer containing 20 mM Hepes, 118 mM NaCl, 4.5 mM KCl, 2.5 mM $CaCl_2$, 1.2 mM $MgSO_4$, pH 7.5 with 2 mg/ml BSA. Radioligand binding was carried out with [$^{125}$I] alpha-bungarotoxin from New England Nuclear, specific activity about 16μuCi/μg, used at 0.4 nM final concentration in a 96 well microtiter plate. The plates were incubated at 37 °C for 3 hours with 25 μl drugs or vehicle for total binding, 100 μl [$^{125}$I] Bungarotoxin and 125 μl tissue preparation. Nonspecific binding was determined in the presence of alpha-bungarotoxin at 1 μM final concentration. The reaction was terminated by filtration using 0.5% Polyethylene imine treated GF/B filters on a Brandel cell harvester with ice-cold buffer, filters were dried overnight, and counted on a Beta plate counter using Betaplate Scint. Data are expressed as IC50's (concentration that inhibits 50% of the specific binding) or as an apparent Ki, IC50/1+[L]/KD. [L] = ligand concentration, KD = affinity constant for [$^{125}$I] ligand determined in separate experiment.

5-$HT_3$ Receptor Binding in NG-108 Cells Using 3H-LY278584: NG-108 cells endogenously express 5-$HT_3$ receptors. Cells are grown in DMEM containing 10% fetal bovine serum supplemented with L-glutamine (1:100). Cells are grown to confluence and harvested by removing the media, rinsing the flasks with phosphate buffered saline (PBS) and then allowed to sit for a 2-3 minutes with PBS containing 5 mM EDTA. Cells are dislodged and poured into a centrifuge tube. Flasks are rinsed with PBS and added to centrifuge tube. The cells are centrifuged for ten minutes at 40,000 x g (20,000 rpm in Sorvall SS34 rotor(Kendro Laboratory Products, Newtown, CT)). The supernatant is discarded (into chlorox) and at this point the remaining pellet is weighed and can be stored frozen (-80 degrees C) until used in the binding assay. Pellets (fresh or frozen - 250 mgs per 96 well plate) are homogenized in 50 mM Tris HCl buffer containing 2 mM $MgCl_2$ (pH 7.4) using a Polytron homogenizer (setting 15,000 rpm) for ten seconds. The homogenate is centrifuged for ten minutes at 40,000 x g. The supernatant is discarded and the pellet resuspended with the Polytron in fresh ice-cold 50 mM Tris HCl containing 2 mM $MgCl_2$ (pH 7.4) buffer and centrifuged again. The final pellet is resuspended in assay buffer (50 mM Tris HCl buffer (pH 7.4 at 37°C degrees) containing 154 mM NaCl,) for a final tissue concentration of 12.5 mg per mL buffer (1.25 X final concentration). Incubations were initiated by the addition of tissue homogenate to 96 well polypropylene plates containing test compounds that have been diluted in 10% DMSO/50 mM Tris buffer and radioligand (1 nM final concentration of 3H-LY278584). Nonspecific binding was determined using a saturating Concentration of a known potent 5-$HT_3$ antagonist (10 uM ICS-205930). After an hour incubation at 37°C in a water bath, the incubation is ended by rapid filtration under vacuum through a fire-treated Whatman GF/B glass fiber filter (presoaked in 0.5% Polyethylene imine for two hours and dried) using a 96 well Skatron Harvester (3 sec pre-wet; 20 seconds wash; 15 seconds dry). Filters are dried overnight and then placed into Wallac sample bags with 10 mLs BetaScint. Radioactivity is quantified by liquid scintillation counting using a BetaPlate counter (Wallac, Gaithersburg, MD). The percent inhibition of specific binding is calculated for each concentration of test compound. An IC50 value (the concentration which inhibits 50% of the specific binding) is determined by linear regression of the concentration-response data (log concentration vs. logit percent values). Ki values are calculated according to Cheng & Prusoff - Ki = IC50/(1 + (L/Kd)), where L is the concentration of the radioligand used in the experiment and the Kd value is the dissociation constant for the radioligand determined in separate saturation experiments.

The following experimental examples illustrate but do not limit the present invention. In the examples, commercial reagents were used without further purification. Purification by chromatography was done on prepacked silica columns from Biotage (Dyax Corp, Biotage Division, Charlottesville, VA). Melting points (mp) were obtained using a Mettler Toledo FP62 melting point apparatus (Mettler-Toledo, Inc., Worthington, OH) with a temperature ramp rate of 10°C/min and are uncorrected. Proton nuclear magnetic resonance ($^1$H NMR) spectra were recorded in deuterated solvents on a Varian INOVA400 (400 MHz) spectrometer (Varian NMR Systems, Palo Alto, CA). Chemical shifts are reported in parts per million (ppm, δ) relative to $Me_4Si$ (δ 0.00). Proton NMR splitting patterns are designated as singlet (s), doublet (d), triplet (t), quartet (q), quintet (quin), sextet (sex), septet (sep), multiplet (m) apparent (ap) and broad (br). Coupling constants are reported in hertz (Hz). Carbon-13 nuclear magnetic resonance ($^{13}$C NMR) spectra were recorded on a Varian INOVA400 (100 MHz). Chemical shifts are reported in ppm (δ) relative to the central line of the 1:1:1 triplet of deuterochloroform (δ 77.00), the center line of deuteromethanol (δ 49.0) or deuterodimethylsulfoxide (δ 39.7). The number of carbon resonance's reported may not match the actual number of carbons in some molecules due to magnetically and chemically equivalent carbons and may exceed the number of actual carbons due to conformational isomers. Mass spectra (MS) were obtained using a Waters ZMD mass spectrometer using flow injection atmospheric pressure chemical ionization (APCI) (Waters Corporation, Milford, Mass). Gas chromatography with mass detection (GCMS) were obtained using a Hewlett Packard HP 6890 series GC system with a HP 5973 mass selective detector and a HP-1 (crosslinked methyl siloxane) column (Agilent Technologies, Wilmington, DE). Room temperature (RT) refers to 20-25 °C. The abbreviations "h" and "hrs" refer to "hours". 1,4-Diaza-bicyclo[3.2.2]nonane was prepared via slight modifications of the published procedure: see, Rubstov, M.V.; Mikhlina, E.E.; Vorob'eva, V. Ya.; Yanina, A. Zh. Obshch. Khim. 1964, V34, 2222-2226.

Reference Example 1

1,4-DIAZA-BICYCLO[3.2.2]NONANE-4-CARBOXYLIC ACID 4-BENZOYL-PHENYL ESTER

Phosgene (1.22 mL, 2.3 mmol, 20% in $PhCH_3$) was slowly added to a solution of 4-hydroxybenzophenone (440 mg, 2.2 mmol) and $Et_3N$ (280 μL, 4.0 mmol) in $PhCH_3$ (10.0 mL) at RT. The mixture was stirred for a period of 3 h. $Et_3N$ (280 μL, 4.0 mmol), polymer supported DMAP (140 mg, 0.2 mmol) and 1,4-diaza-bicyclo[3.2.2]nonane (256 mg, 2.0 mmol) were added. The mixture was allowed to stir for 2 h. at RT and then was heated to 100 °C for 16 h. The reaction mixture was allowed to cool to RT, filtered and $CHCl_3$ (40 mL) was added. The organics were washed with $H_2O$ (10 mL x 2) and brine (10 mL) and then dried ($Na_2SO_4$), filtered and concentrated. The crude residue was purified by chromatography (Biotage 40M column) eluting with 5% MeOH in $CHCl_3$ containing 20 drops of $NH_4OH$ per liter of eluent to afford 116 mg (15% yield) of the title compound as a white solid: $^1H$ NMR ($CDCl_3$, 400 MHz, mixture of conformational isomers) δ 7.83 (d, 2H, J = 8.7 Hz), 7.78 (d, 2H, J = 7.5 Hz), 7.57 (t, 1H, J = 7.5 Hz), 7.47 (t, 2H, J = 7.5 Hz), 7.26-7.22 (m, 2H), 4.48-4.47 (m, 1H, major), 4.41-4.40 (m, minor), 3.86 (t, J = 5.8 Hz, minor), 3.78 (t, 2H, J = 5.8 Hz), 3.20-3.02 (m, 6H), 2.14-2.08 (m, 2H), 1.83-1.73 (m, 2H); $^{13}C$ NMR ($CDCl_3$, 100 MHz) δ 195.9, 155.0, 154.9, 153.4, 152.6, 137.9, 134.7, 134.6, 132.6, 131.84, 131.81, 130.2, 128.5, 121.74, 121.72, 57.4, 57.1, 49.2, 49.1, 46.5, 46.4, 43.2, 42.9, 27.4, 26.6; MS (Cl) m/z 351.3 (M + H). The hydrochloride salt was prepared by dissolving the title compound in ethyl acetate and adding 3N HCl in ethyl acetate; m.p. = 236. °C.

Unless otherwise indicated, the procedure described in Example 1 was used to prepare the title compounds of Examples 2 through 8.

Example 2

1,4-DIAZA-BICYCLO[3.2.2]NONANE-4-CARBOXYLIC ACID 4-IMIDAZOL-1-YL-PHENYL ESTER

1-(4-Hydroxyphenyl)imidazole was used. The title compound was prepared in 27% yield as an oil: $^1H$ NMR ($CDCl_3$, 400 MHz, mixture of conformational isomers) δ 7.77 (s, 1H), 7.35 (d, 2H, J = 8.7 Hz), 7.23-7.18 (m. 4H), 4.45-4.44 (m, 1H, major), 4.37-4.35 (m, minor), 3.83 (t, J = 5.8 Hz, minor), 3.74 (t, 2H, J = 5.8 Hz, major), 3.16-2.97 (m, 6H), 2.12-2.02 (m, 2H), 1.81-1.70 (m, 2H); $^{13}C$ NMR ($CDCl_3$, 100 MHz) δ 153.8, 153.0, 150.8, 150.7, 135.9, 134.7, 134.6, 130.5, 123.4, 122.81. 122.77, 118.7, 57.4, 57.1, 49.2, 49.1, 46.44, 46.37, 43.3, 43.0, 27.4, 26.7; MS (Cl) m/z 313.3 (M + H). The dihydrochloride salt was prepared; m.p. > 300°C.

Example 3

1,4-DIAZA-BICYCLO[3.2.2]NONANE-4-CARBOXYLIC ACID 4-[1,2,4]TRIAZOL-1-YL-PHENYL ESTER

4-(1-H-1,2,4-triazol-1-yl)phenol was used. The title compound was prepared in 13% yield as a colorless oil: $^1H$ NMR ($CDCl_3$, 400 MHz, mixture of conformational isomers) δ 8.51 (s, 1H), 8.08 (s, 1H), 7.65 (d, 2H, J = 8.4 Hz), 7.27-7.23 (m, 2H), 4.46-4.44 (m, 1H, major), 4.38-4.36 (m, minor), 3.84 (t, J = 5.8 Hz, minor), 3.75 (t, 2H, J = 5.8 Hz, major), 3.18-2.98 (m, 6H), 2.13-2.03 (m, 2H), 1.81-1.71 (m, 2H); $^{13}C$ NMR ($CDCl_3$, 100 MHz) δ 153.7, 152.8, 151.3, 151.2, 141.2, 134.3, 134.2, 123.4, 121.32, 121.28, 57.4, 57.1, 49.3, 49.1, 46.5, 46.4, 43.3, 43.0, 27.5, 26.7; MS (Cl) m/z 314.3 (M + H). The hydrochloride salt was prepared; m.p. = 253.1°C.

Example 4

1,4-DIAZA-BICYCLO[3.2.2]NONANE-4-CARBOXYLIC ACID 4-(4-ACETYL-PIPERAZIN-1-YL)-PHENYL ESTER

1-Acetyl-4-(4-hydroxyphenyl)piperazine was used. The title compound was prepared in 44% yield as a yellow oil: $^1H$ NMR ($CDCl_3$, 400 MHz, mixture of conformational isomers) δ 6.99-6.96 (m, 2H), 6.88-6.84 (m 2H), 4.41-4.40 (m, 1H, major), 4.32-4.31 (m, minor), 3.80-3.55 (m, 10H), 3.11-2.94 (m, 6H), 2.08 (s, 3H), 2.08-2.02 (m, 2H), 1.76-1.65 (m, 2H): $^{13}C$ NMR ($CDCl_3$, 100 MHz) δ 169.5, 154.6, 153.8, 148.8, 148.7, 145.4, 145.3, 122.5, 117.9, 58.2, 57.3, 57.1, 50.6, 50.5, 50.1, 48.9, 48-7, 46.4, 46.3, 46.2, 42.9, 42.6, 41.6, 27.2, 26.5, 21.5, 18.5; MS (Cl) m/z 373.4 (M + H). The dihydrochloride salt was prepared; m.p. = 166.6 °C.

Example 42

1

Example 5

1,4-DIAZA-BICYCLO[3.2.2]NONANE-4-CARBOXYLIC ACID 2-BENZOOXAZOL-2-YL-PHENYL ESTER

2-(o-Hydroxyphenyl)benzoxazole was used. The title compound was prepared in 35% yield as a yellow oil: $^1H$ NMR ($CDCl_3$, 400 MHz, mixture of conformational isomers) δ 8.22-8.19 (m, 1H), 7.73-7.66 (m, 1H), 7.55-7.42 (m, 2H), 7.41-7.30 (m, 4H), 4.71-4.69 (m, 1H, major), 4.33-4.30 (m, minor), 3.99 (t, J = 5.8 Hz, minor), 3.74 (t, 2H, J = 5.8 Hz, major), 3.4-2.7 (m, 6H), 2.35-2.00 (m, 2H), 1.81-1.68 (m, 2H); MS (Cl) m/z 364.2 (M + H). The hydrochloride salt was prepared; m.p. = 259.9°C.

Example 6

1,4-DIAZA-BICYCLO[3.2.2]NONANE-4-CARBOXYLIC ACID 2-BENZOTHIAZOL-2-YL-PHENYL ESTER

2-(2-Hydroxyphenyl)benzothiazole was used. The title compound was prepared in 23% yield as a yellow oil: $^1H$ NMR ($CDCl_3$, 400 MHz, mixture of conformational isomers) δ 8.26-7.90 (m, 4H), 7.52-7.47 (m 1H), 7.42-7.34 (m, 2H), 7.27-7.22 (m, 1H), 4.74-4.73 (m, minor), 4.41-3-39 (m, 1H, major), 4.04 (t, 2H, J = 5.8 Hz, major), 3.79 (t, J = 5.8 Hz, minor), 3.28-3.05 (m, 6H), 2.27-2.02 (m, 2H), 1.94-1.72 (m, 2H); MS (Cl) m/z 380.2 (M + H). The hydrochloride salt was prepared; m.p. = 247.6°C.

Example 7

1,4-DIAZA-BICYCLO[3.2.2]NONANE-4-CARBOXYLIC ACID 3-PYRIDIN-3-YL-PHENYL ESTER

3-Pyridin-3-yl-phenol (see below for preparation) was used. The title compound was prepared in 29% yield as a white solid: $^1H$ NMR ($CDCl_3$, 400 MHz, mixture of conformational isomers) δ 8.81 (d, 1H, J = 1.6 Hz), 8.57 (dd, 1H, J = 5.0, 1.6 Hz), 7.86-7.83 (m, 1H), 7.47-7.32 (m, 4H), 7.16-7.14 (m, 1H), 4.47-4.46 (m, 1H, major), 4.38-4.37 (m, minor), 3.84 (t, J = 5.8 Hz, minor), 3.75 (t, 2H, J = 5.8 Hz, major), 3.16-2.95 (m, 6H), 2.14-2.02 (m, 2H), 1.80-1.66 (m, 2H); $^{13}C$ NMR ($CDCl_3$, 100 MHz) δ 154.0, 153.3, 152.3, 152.2, 149.0, 148.5, 139.4, 139.3, 136.0, 134.7, 130.2, 130.1, 124.3, 124.2, 123.8, 121.7, 120.9, 57.6, 57.2, 49.2, 49.1, 46.5, 46.4, 43.4, 43.1, 27.6, 26.8; MS (Cl) m/z 324.3 (M + H). The dihydrochloride salt was prepared; m.p. = 210.2°C.

Example 8

3-PYRIDIN-3-YL-PHENOL HYDROBROMIDE

3-Methoxyphenylboronic acid (0.334 g, 2.2 mmol), sodium carbonate (0.848 g, 8.0 mmol) and tetrakistriphenylphosphine palladium (0.231 g, 0.2 mmol) were added to a flask and the flask was purged with nitrogen. Ethanol (30.0 mL) and water (1.5 mL) were added followed by 3-bromopyridine (0.316 g, 2.0 mmol). The reaction mixture was heated to 80°C for a period of 8 h. After cooling to RT, the mixture was diluted with water (5.0 mL) and extracted with ethyl acetate (20 mL x 4). The combined organic extracts were washed with brine (25 mL), dried ($Na_2CO_3$), filtered and concentrated. The crude residue was purified by chromatography (Biotage, 40S) eluting with 10% ethyl acetate in hexanes to afford 239 mg (65%) of 3-(3-Methoxy-phenyl)-pyridine as a yellow oil: MS (Cl) m/z 186.1 (M + H).

The 3-(3-Methoxy-phenyl)-pyridine was treated with HBr (5 mL) at 100°C for 12 h. The reaction mixture was allowed to cool to RT and concentrated to afford 298 mg (92%) of the title compound as a white solid: $^1H$ NMR ($CDCl_3$, 400 MHz, mixture of conformational isomers) δ 9.14 (d, 1H, J = 2.1 Hz), 8.90-8.83 (m, 2H), 8.17 (dd, 1H, J = 8.3, 5.8 Hz), 7.39 (t, 1H, J = 8.1 Hz), 7.27-7.25 (m. 1H), 7.20-7.19 (m, 1H), 6.98-6.95 (m, 1H), 4.93 (br s, 1H); $^{13}C$ NMR ($CDCl_3$, 100 MHz) δ 158.7, 144.6, 140.9, 139.7, 139.6, 134.9, 130.8, 127.7, 118.3, 117.1, 114.0; GCMS m/z 171 (M).

Reference Example 9

1,4-DIAZA-BICYCLO[3.2.2]NONANE-4-CARBOXYLIC ACID BIPHENYL-4-YL ESTER

1,4-Diaza-bicyclo[3.2.2]nonane-4-carboxylic acid 4-(4,4,5,5-tetramethyl[1,3,2]-dioxa-borolan-2-yl)-phenyl ester (76.0 mg, 0.204 mmol), bromobenzene (43.0 μL, 0.408 mmol), [1,1'-bis(diphenylphosphino)ferrocene] dichloropalladium (II) dichloromethane adduct (16.7 mg, 0.0204 mmol), 1,1'-bis(diphenylphosphino)ferrocene (11.3 mg, 0.0204 mmol), and potassium phosphate (130 mg, 0.612 mmol) were added to a flask and purged with a stream of nitrogen. 1,4-Dioxane (2.46 mL) and water (122 μL) were added and the mixture was placed in an oil bath at 80°C for 20 h. The reaction mixture was allowed to cool to RT, diluted with ethyl acetate (5 mL) and water (5 mL). The layers were partitioned and the aqueous layer was extracted with ethyl acetate (5 mL x 3). The combined organic layers were washed with water (10 mL x 2), brine (10 mL) and dried ($Na_2CO_3$). After filtration and concentration, the crude residue was purified by chromatography (Biotage, 12M, 4% methanol/chloroform to 6% methanol/chloroform gradient) to afford 41.6 mg (63%) of the title compound as a brown oil: $^1$H NMR ($CDCl_3$, 400 MHz, mixture of conformational isomers) δ 7.59-7.55 (m, 4H), 7.43 (t, 2H, J = 7.9 Hz), 7.34 (t, 1H, J = 7.5 Hz), 7.22-7.17 (m, 2H), 4.48-4.47 (m, 1H, major), 4.41-4.39 (m, minor), 3.85 (t, J = 5.8 Hz, minor), 3.77 (t, 2H, J = 5.8 Hz, major), 3.19-3.00 (m, 6H), 2.16-2.05 (m, 2H), 1.81-1.70 (m, 2H); $^{13}$C NMR ($CDCl_3$, 100 MHz) δ 154.2, 153.4, 151.2, 151.1, 140.7, 138.6, 138.5, 129.0, 128.3, 128.2, 127.5, 127.3, 123.8, 122.3, 57.6, 57.3, 49.1, 49.0, 46.6, 46.5, 43.3, 42.9, 27.5, 26.7; GCMS m/z 322 (M). The hydrochloride salt was prepared by dissolving the title compound in ethyl acetate and adding 3N HCl in ethyl acetate.

Unless otherwise indicated, procedures analogous to the procedure described in Example 9 were used to prepare the title compounds of Examples 10 through 12.

Example 10

1,4-DIAZA-BICYCLO[3.2.2]NONANE-4-CARBOXYLIC ACID 4-PYRIDIN-2-YL-PHENYL ESTER

2-Bromopyridine was used. The title compound was prepared in 60% yield as a brown oil: $^1$H NMR ($CDCl_3$, 400 MHz, mixture of conformational isomers) δ 8.65 (d, 1H, J = 4.6 Hz), 7.98 (d, 2H, J = 8.7 Hz), 7.74-7.67 (m, 2H), 7.23-7.16 (m, 3H), 4.47-4.45 (m, 1H, major), 4.39-4.38 (m, minor), 3.84 (t, J = 5.8 Hz, minor), 3.75 (t, 2H, J = 5.8 Hz, major), 3.16-2.99 (m, 6H), 2.13-2.06 (m, 2H), 1.80-1.68 (m, 2H); $^{13}$C NMR ($CDCl_3$, 100 MHz) δ 156.9, 154.0, 153.2, 152.4, 152.4, 149.9, 137.0, 136.7, 136.6, 131.5, 131.4, 128.6, 128.5, 128.12, 128.09, 122.3, 122.21, 122.19, 120.6. 57.5, 57.2, 49.1, 49.0, 46.5, 46.4, 43.3, 42.9, 27.5, 26.7; MS (Cl) m/z 324.3 (M + H). The dihydrochloride salt was prepared.

Example 11

1,4-DIAZA-BICYCLO[3.2.2]NONANE-4-CARBOXYLIC ACID 4-PYRIDIN-3-YL-PHENYL ESTER

3-Bromopyridine was used. The title compound was prepared in 72% yield as a brown oil: $^1$H NMR ($CDCl_3$, 400 MHz, mixture of conformational isomers) δ 8.79 (s, 1H), 8.55 (d, 1H, J = 4.6 Hz), 7.81 (d, 1H, J = 8.3 Hz), 7.54 (d, 2H, J = 8.3 Hz), 7.32 (dd, 1H, J = 7.9, 5.0 Hz), 7.21 (dd, 2H, J = 8.3, 3.0 Hz), 4.46-4.45 (m, 1H, major), 4.37-4.36 (m, minor), 3.83 (t, J = 5.8 Hz, minor), 3.74 (t, 2H, J = 5.8 Hz, major), 3.16-2.98 (m, 6H), 2.11-2.02 (m, 2H), 1.78-1.67 (m, 2H); $^{13}$C NMR ($CDCl_3$, 100 MHz) δ 154.0, 153.2, 151.8, 151.7, 148.6, 148.4, 136.2, 135.1, 135.0, 134.5, 131.5, 131.4, 128.6, 128.5, 128.3, 128.2, 123.8, 122.7, 57.5, 57.2, 49.2, 49.1, 46.5, 46.4, 43.3, 43.0, 27.5, 26.7; MS (Cl) m/z 324.3 (M + H). The dihydrochloride salt was prepared.

Example 12

1,4-DIAZA-BICYCLO[3.2.2]NONANE-4-CARBOXYLIC ACID 4-PYRIDIN-4-YL-PHENYL ESTER

4-Bromopyridine hydrochloride was used. The title compound was prepared in 49% yield as a brown oil: $^1$H NMR ($CDCl_3$, 400 MHz, mixture of conformational isomers) δ 8.61 (br s, 2H), 7.61 (d, 2H, J = 8.3 Hz), 7.45 (d, 2H, J = 5.0 Hz), 7.23 (dd, 2H, J = 8.3, 3.5 Hz), 4.46-4.45 (m, 1H, major), 4.37-4.36 (m, minor), 3.83 (t, J = 5.8 Hz, minor), 3.74 (t, 2H, J = 5.8 Hz, major), 3.16-2.98 (m, 6H), 2.11-2.02 (m, 2H), 1.79-1.69 (m, 2H); $^{13}$C NMR ($CDCl_3$, 100 MHz) δ 153.9, 153.1, 152.5, 152.4, 150.5, 147.8, 135.4, 135.3, 131.5, 131.4, 128.6, 128.5, 128.22, 128.17, 122.7, 121.8, 57.5, 57.2, 49.2, 49.1, 46.5, 46.4, 43.4, 43.0, 27.5, 26.7; MS (Cl) m/z 324.3 (M + H). The dihydrochloride salt was prepared.

The following slightly modified procedure was used to prepare the title compounds of Examples 13 through 22.

RAM tubes were charged with aryl bromides (0.125 mmol). A solution of 1,1'-bis(diphenylphosphino)ferrocene in dioxane (2.772 mg per 0.2 mL) and a solution of 1,4-diaza-bicyclo[3.2.2]nonane-4-carboxylic acid 4-(4,4,5,5-tetramethyl[1,3,2]dioxaboro-lan-2-yl)-phenyl ester in dioxane (18.6 mg per 0.7 mL dioxane) were added to each reaction tube. Next a solution of $K_3PO_4$ in $H_2O$ (33.1 mg per 0.05 mL) were added with stirring. Finally, [1,1'-bis(diphenylphosphino) ferrocene] dichloropalladium (II) dichloromethane adduct was added as a slurry in DMF (4.08 mg per 0.05 mL). The reactions were heated at 95 °C overnight under argon with shaking to agitate. The reaction mixtures were worked up by adding water (2 mL), followed by EtOAc (4 mL, sip and spit agitation). Remove organic layer (top) and pass through

an SPE cartridge with $Na_2SO_4$. Re-extract reaction with 3 ml EtOAc, then 2 ml EtOAc and combine organic extracts and concentrate. The crude residue was purified by reverse phase HPLC using a Micromass Platform LC System with a Waters Symmetry $C_{18}$, 5 μm, 30 x 150 mm column using gradient elution. Solvent A is 0.1% trifluoroacetic acid in water and Solvent B is acetonitrile. The flow rate was 20 mL/min. A linear gradient of 0-100% B over 15 min. was used and the products were collected by mass trigger (ES+) and concentrated in a GeneVac. The products were analyzed by analytical HPLC using a Waters Alliance System with a Waters Symmetry $C_{18}$, , 5 μm, 2.1 x 150 mm column using gradient elution. The flow rate was 0.5 mL/min. Two different gradients using the solvent systems described above were used. Method 1 (M1) used a linear gradient of 0-100% B over 10 min. Method 2 (M2) used a linear gradient of 10-100% B over 10 min.

Example 13

1,4-DIAZA-BICYCLO[3.2.2]NONANE-4-CARBOXYLIC ACID 4-FURAN-3-YL-PHENYL ESTER

3-Bromofuran was used. The title compound was prepared In 5.2% yield as it's trifluoroacetic acid salt: MS (ES+) m/z 313.0 (M + H), HPLC retention time (M1) = 6.330 min.

Example 14

1,4-DIAZA-BICYCLO[3.2.2]NONANE-4-CARBOXYLIC ACID 4-(6-FLUORO-PYRIDIN 3-YL-PHENYL ESTER

5-Bromo-2-fluoropyridine was used. The title compound was prepared in 53.1% yield as it's bistrifluoroacetic acid salt: MS (ES+) m/z 342.0 (M + H). HPLC retention time (M1) = 6.011 min.

Example 15

1,4-DIAZA-BICYCLO[3.2.2]NONANE-4-CARBOXYLIC ACID 4-(5-ETHOXYCARBONYL PYRIDIN-3-YL)-PHENYL ESTER

5-Bromo-nicotinic acid ethyl ester was used. The title compound was prepared in 30.5% yield as it's bistrifluoroacetic acid salt: MS (ES+) m/z 396.0 (M + H), HPLC retention time (M2) = 4.981 min.

Example 16

1,4-DIAZA-BICYCLO[3.2.2]NONANE-4-CARBOXYLIC ACID 4-QUINOLIN-3-YL-PHENYL ESTER

3-Bromoquinoline was used. The title compound was prepared in 28.6% yield as it's bistrifluoroacetic acid salt: MS (ES+) m/z 374.0 (M + H), HPLC retention time (M2) = 4.219 min.

Example 17

1,4-DIAZA-BICYCLO[3,2,2]NONANE-4-CARBOXYLIC ACID 4-(6-METHYL-PYRIDIN-2-YL)-PHENYL ESTER

2-Bromo-6-methylpyridine was used. The title compound was prepared in 35.4% yield as it's bistrifluoroacetic acid salt: MS (ES+) m/z 338.0 (M + H), HPLC retention time (M2) = 2.086 min.

Example 18

1,4-DIAZA-BICYLO[3.2.2]NONANE-4-CARBOXYLIC ACID 4-(3,5-DIMETHYL-ISOXAZOL-4-YL)-PHENYL ESTER

4-Bromo-3,5-dimethylisoxazole was used. The title compound was prepared in 17.6% yield as it's trifluoroacetic acid salt: MS (ES+) m/z 342.0 (M + H), HPLC retention time (M2) = 5.076 min.

Example 19

1,4-DIAZA-BICYCLO[3.2.2]NONANE-4-CARBOXYLIC ACID 4-(4-METHYL-PYRIDIN-2-YL)-PHENYL ESTER

2-Bromo-4-methylpyridine was used. The title compound was prepared in 27.6% yield as it's bistrifluoroacetic

acid salt: MS (ES+) m/z 338.0 (M + H), HPLC retention time (M2) = 2.805 min.

Example 20

1,4-DIAZA-BICYCLO[3.2.2]NONANE-4-CARBOXYLIC ACID 4-(5-CARBAMOYL-PYRIDIN-3-YL)-PHENYL ESTER

5-Bromo-nicotinamide was used. The title compound was prepared in 20.9% yield as it's bistrifluoroacetic acid salt: MS (ES+) m/z 367.0 (M + H), HPLC retention time (M2) = 2.125 min.

Example 21

1,4-DIAZA-BICYCLO[3.2.2]NONANE-4-CARBOXYLIC ACID 4-(5-CYANO-PYRIDIN-3-YL)-PHENYL ESTER

5-Bromopyridine-3-carbonitrile was used. The title compound was prepared in 45.8% yield as it's bistrifluoroacetic acid salt: MS (ES+) m/z 349.0 (M + H), HPLC retention time (M2) = 4.832 min.

Example 22

1,4-DIAZA-BICYCLO[3.2.2]NONANE-4-CARBOXYLIC ACID 4-IMIDAZO[1,2-A]PYRIDIN-3-YL-PHENYL ESTER

3-Bromo-imidazo[1,2-a]pyridine was used. The title compound was prepared in 45.8% yield as it's trifluoroacetic acid salt MS (ES+) m/z 363.0 (M + H), HPLC retention time (M2) = 2.771 min.

## Claims

**1.** A compound of the formula

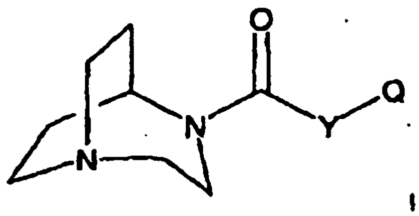

wherein Y = O or NH;

Q is $C_6$-aryl, which is substituted with $R^3$;
$R^3$ is (5-12 membered) heteroaryl that is optionally substituted with from one to five substituents independently selected from H, F, Cl, Br, I, nitro, cyano, $CF_3$, $-NR^6R^7$, $-NR^6C(=O)R^7$, $-NR^6C(=O)NR^7R^8$, $-NR^6S(=O)_2R^7$, $-NR^6S(=O)_2NR^7R^8$, $-OR^6$, $-OC(=O)R^6$, $-OC(=O)OR^8$, $-OC(=O)NR^6R^7$, $-OC(=O)SR^6$, $-C(=O)OR^6$, $-C(=O)R^8$, $-C(=O)NR^6R^7$, $-SR^6$, $-S(=O)R^6$, $-S(=O)_2R^6$, $-S(=O)_2NR^6R^7$, straight chain or branched ($C_1$-$C_6$)alkyl, straight chain or branched ($C_2$-$C_6$)alkenyl, straight chain or branched ($C_2$-$C_8$)alkynyl, ($C_3$-$C_8$)cycloalkyl, ($C_4$-$C_8$)cycloalkenyl, (3-8 membered) heterocycloalkyl, ($C_8$-$C_{11}$)bicycloalkyl, ($C_7$-$C_{11}$)bicycloalkenyl, (5-11 membered) heterobicycloalkyl, (5-11 membered) heterobicycloalkenyl, ($C_8$-$C_{11}$) aryl, (5-12 membered) heteroaryl, and $R^6$;
each $R^6$, $R^7$, and $R^8$ is independently selected from H, straight chain or branched ($C_3$-$C_6$)alkyl, straight chain of branched ($C_2$-$C_6$)alkenyl, straight chain or branched ($C_2$-$C_6$)alkynyl, ($C_3$-$C_8$)cycloalkyl, ($C_4$-$C_8$)cycloalkenyl, 3-8 membered heterocycloalkyl, ($C_5$-$C_{11}$)bicycloakly, ($C_7$-$C_{15}$)bicycloalkenyl, 5-11 membered heterobicycloalkyl, 5-11 membered heterobicycloalkenyl, ($C_8$-$C_{11}$) aryl and (5-12 membered heteroaryl; wherein $R^6$, $R^7$, and $R^8$ are each independently optionally substituted with from one to six substituents, independently selected from F, Cl, Br, I, nitro, cyano, $CF_3$, $-NR^9R^{10}$, $-NR^9C(=O)R^{10}$, $-NR^9C(=O)NR^{10}R^{11}$, $-R^9S(=O)_2R^{10}$, $-NR^9S(=O)_2NR^{10}R^{11}$, $-OR^9$, $-OC(=O)R^9$, $-OC(=O)OR^9$, $-OC(=O)NR^9R^{10}$, $-OC(=O)SR^9$, $-C(=O)OR^9$, $-C(=O)R^9$, $-C(=O)NR^6R^7$, $-SR^8$, $-S(=O)R^8$, $-S(=O)_2R^8$, $-S(=O)_2NR^6R^7$, straight chain or branched ($C_1$-$C_6$)alkyl, straight chain or branched ($C_2$-$C_8$)alkenyl, straight chain or branched ($C_2$-$C_8$)alkynyl, ($C_3$-$C_8$)cycloalkyl, ($C_4$-$C_8$)cycloalkenyl, 3-8 membered heterocycloalkyl, ($C_5$-$C_{11}$)bicycloalkyl, ($C_7$-$C_{11}$)bicycloalkenyl, 5-11 membered heterobicycloalkyl, (5-11 membered) heterobicycloalkenyl, ($C_8$-$C_{11}$) aryl, 5-12 membered heteroaryl, and $R^9$;

each $R^9$, $R^{10}$, and $R^{11}$ is independently selected from H, straight chain or branched $(C_1-C_8)$alkyl, straight chain or branched $(C_2-C_6)$alkenyl, straight chain or branched $(C_2-C_8)$alkynyl, $(C_3-C_8)$cycloalkyl, $(C_4-C_8)$cycloalkenyl, 3-8 membered heterocycloalkyl, $(C_5-C_{11})$bicycloalkyl, $(C_7-C_{11})$bicycloalkenyl, (5-11 membered heterobicycloalkyl, 5-11 membered heterobicycloalkenyl, $(C_6-C_{11})$ aryl and 5-12 membered heteroaryl;

or an enantiomeric, diastereomeric, and tautomeric isomer of such compound, or a pharmaceutically acceptable salt of such compound or isomer.

**2.** A compound according to claim 1 wherein Y = O.

**3.** A compound according to claim 1 that is selected from the following compounds and their pharmaceutically acceptable salts:

1,4-Diaza-bicyclo[3.2.2]nonane-4-carboxylic acid 4-pyridn-2-yl-phenyl ester;
1,4-Diaza-bicyclo[3.2.2]nonane-4-carboxylic acid 4-pyridin-3-yl-phenyl ester;
1,4-Diaza-bicyclo[3.2.2]nonane-4-carboxylic acid 4-pyridin-4-yl-phenyl ester,
1,4-Diaza-bicyclo[3.2.2]nonane-4-carboxylic acid 4-furan-3-yl-phenyl ester,
1,4-Diaze-bicyclo[3.2.2]nonane-4-carboxylic acid 4-(6-fluoro-pyridin-3-yl)-phonyl ester;
1,4-Diaza-bicyclo[3.2.2]nonane-4-carboxylic acid 4-imidazol-1-yl-phenyl ester,
1,4-Diaza-bicyclo[3.2.2]nonane-4-carboxylic acid 4-[1,2,4]triazol-1-yl-phenyl ester;
1,4-Diaza-bicyclo[3.2.2]nonane-4-carboxylic acid 2-benzooxazol-2-yl-phenyl ester;
1,4-Diaza-bicyclo[3.2.2]nonane-4-carboxylic acid 2-benzothiazol-2-yl-phenyl ester;
1,4-Diaza-bicyclo[3.2.2]nonane-4-carboxylic acid 4-(5-ethoxycarbanyl-pyridin-3-yl)-phenyl ester;
1,4-Diaza-bicyclo[3.2.2]nonane-4-carboxylic acid 4-(6-methyl-pyridin-2-yl)-phenyl ester;
1,4-Diaza-bicyclo[3.2.2]nonane-4-carboxylic acid 4-(3,5-dimethyl-isoxazol-4-yl)-phenyl ester;
1,4-Diaza-bicyclo[3.2.2]nonane-4-carboxylic acid 4-(4-methyl-pyridln-2-yl)-phenyl ester;
1,4-Diaza-bicyclo[3.2.2]nonane-4-carboxylic acid 4-(5-carbamoyl-pyridin-3-yl)-phenyl ester;
1,4-Diaza-bicyclo[3.2.2]nonane-4-carboxylic acid 4-(5-cyano-pyridin-3-yl)-phenyl ester;
1,4-Diaza-bicyclo[3.2.2]nonane-4-carboxylic acid 4-imidazo[1,2-a]pyridin-3-yl-phenyl ester; and
1,4-Diaza-bicyclo[3.2.2]nonane-4-carboxylic acid 3-pyridin-3-yl-phenyl ester.

**4.** A pharmaceutical composition comprising a compound according to claim 1 and a pharmaceutically acceptable carrier.

**5.** A compound according to claim 1 for use in medicine.

**6.** Use of a compound according to claim 1 in the preparation of a medicament for the treatment of schizophrenia in a mammal.

**7.** Use of a compound according to claim 1 in the preparation of a medicament for the treatment of a disorder or condition selected from inflammatory bowel disease, ulcerative colitis, pyoderma gangrenosum, Crohn's disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotropic lateral sclerosis (ALS), cognitive dysfunction, tinnitus, hypertension, bulimia, anorexia, obesity, cardiac arrythmias, gastric acid hypersecretion, ulcers, pheochromocytoma, progressive supramuscular palsy, chemical dependencies and addictions, dependencies on, or addictions to nicotine (and/or tobacco products), alcohol, benzodiazepines, barbituates, opioids or cocaine, headache, stroke, traumatic brain injury (TBI), psychosis, Huntington's Chorea, tardive dyskinesia, hyperkinesia, dyslexia, multi-infarct dementia, age related cognitive decline, epilepsy, including petit mal absence epilepsy, HIV induced dementia, senile dementia of the Alzheimer's type (AD), Parkinson's disease (PD), attention deficit hyperactivity disorder (ADHD) and Tourette's Syndrome in a mammal.

**8.** A compound according to either claim 1 or claim 2 wherein Q is optionally substituted with one to six substituents independently selected from the substituents exemplified by the examples of claim 3.

## Patentansprüche

**1.** Verbindung der Formel

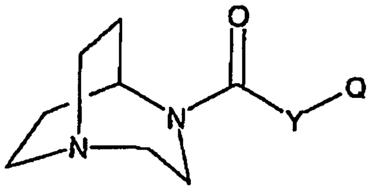

I

worin Y = O oder NH;

Q $C_6$-Aryl ist, das mit $R^3$ substituiert ist;

$R^3$ (5-12-gliedriges) Heteroaryl ist, das wahlweise substituiert ist mit einem bis fünf Substituenten, unabhängig ausgewählt aus H, F, Cl, Br, I, Nitro, Cyano, $CF_3$, $-NR^6R^7$, $-NR^6C(=O)R^7$, $-NR^6C(=O)NR^7R^8$, $-NR^6S(=O)_2R^7$, $-NR^6S(=O)_2NR^7R^8$, $-OR^6$, $-OC(=O)R^6$, $-OC(=O)OR^6$, $-OC(=O)NR^6R^7$, $-OC(=O)SR^6$, $-C(=O)OR^6$, $-C(=O)R^6$, $-C(=O)NR^6R^7$, $-SR^6$, $-S(=O)R^6$, $-S(=O)_2R^6$, $-S(=O)_2NR^6R^7$, geradkettigem oder verzweigtem ($C_1$-$C_8$)Alkyl, geradkettigem oder verzweigtem ($C_2$-$C_8$)Alkenyl, geradkettigem oder verzweigtem ($C_2$-$C_8$)Alkinyl, ($C_3$-$C_8$)Cycloalkyl, ($C_4$-$C_8$)Cycloalkenyl, (3-8-gliedrigem) Heterocycloalkyl, ($C_5$-$C_{11}$) Bicycloalkyl, ($C_7$-$C_{11}$)Bicycloalkenyl, (5-11-gliedrigem) Heterobicycloalkyl, (5-11-gliedrigem) Heterobicycloalkenyl, ($C_6$-$C_{11}$)Aryl, (5-12-gliedrigem) Heteroaryl und $R^6$;

jedes $R^6$, $R^7$ und $R^8$ unabhängig ausgewählt ist aus H, geradkettigem oder verzweigtem ($C_1$-$C_8$)Alkyl, geradkettigem oder verzweigtem ($C_2$-$C_8$)Alkenyl, geradkettigem oder verzweigtem ($C_2$-$C_8$)Alkinyl, ($C_3$-$C_8$)Cycloalkyl, ($C_4$-$C_8$)Cycloalkenyl, 3-8-gliedrigem Heterocycloalkyl, ($C_5$-$C_{11}$)Bicycloalkyl, ($C_7$-$C_{11}$)Bicycloalkenyl, 5-11-gliedrigem Heterobicycloalkyl, 5-11-gliedrigem Heterobicycloalkenyl, ($C_6$-$C_{11}$)Aryl und (5-12-gliedrigem) Heteroaryl;

wobei $R^6$, $R^7$ und $R^8$ jeweils unabhängig wahlweise substituiert sind mit einem bis sechs Substituenten, unabhängig ausgewählt aus F, Cl, Br, I, Nitro, Cyano , $CF_3$, $-NR^9R^{10}$, $-NR^9C(=O)R^{10}$, $-NR^9C(=O)NR^{10}R^{11}$, $-R^9S(=O)_2R^{10}$, $-NR^9S(=O)_2NR^{10}R^{11}$, $-OR^9$, $-OC(=O)R^9$, $-OC(=O)OR^9$ , $-OC(=O)NR^9R^{10}$ , $-OC(=O)SR^9$, $-C(=O)OR^9$, $-C(=O)R^9$, $-C(=O)NR^6R^7$, $-SR^6$, $-S(=O)R^6$, $-S(=O)_2R^6$, $-S(=O)_2NR^6R^7$, geradkettigem oder verzweigtem ($C_1$-$C_8$) Alkyl, geradkettigem oder verzweigtem ($C_2$-$C_8$)Alkenyl, geradkettigem oder verzweigtem ($C_2$-$C_8$)Alkenyl, ($C_3$-$C_8$)Cycloalkyl, ($C_4$-$C_8$)Cycloalkenyl, 3-8-gliedrigem Heterocycloalkyl, ($C_5$-$C_{11}$) Bicycloalkyl, ($C_7$-$C_{11}$)Bicycloalkenyl, 5-11-gliedrigem Heterobicycloalkyl, (5-11-gliedrigem) Heterobicycloalkenyl, ($C_6$-$C_{11}$)Aryl, 5-12-gliedrigem Heteroaryl und $R^9$;

jedes $R^9$, $R^{10}$ und $R^{11}$ unabhängig ausgewählt ist ausgewählt ist aus H, geradkettigem oder verzweigtem ($C_1$-$C_8$)Alkyl, geradkettigem oder verzweigtem ($C_2$-$C_8$)Alkenyl, geradkettigem oder verzweigtem ($C_2$-$C_8$)Alkinyl, ($C_3$-$C_8$)Cycloalkyl, ($C_4$-$C_8$)Cycloalkenyl, 3-8-gliedrigem Heterocycloalkyl, ($C_5$-$C_{11}$) Bicycloalkyl, ($C_7$-$C_{11}$)Bicycloalkenyl, 5-11-gliedrigem Heterobicycloalkyl, 5-11-gliedrigem Heterobicycloalkenyl, ($C_6$-$C_{11}$)Aryl und 5-11-gliedrigem Heteroaryl;

oder ein enantiomeres, diastereomeres und tautomeres Isomer einer solchen Verbindung oder ein pharmazeutisch verträgliches Salz der derartigen Verbindung oder des derartigen Isomers.

**2.** Verbindung nach Anspruch 1, worin Y=O.

**3.** Verbindung nach Anspruch 1, die ausgewählt ist aus den nachstehenden Verbindungen und ihren pharmazeutisch verträglichen Salzen:

1,4-Diaza-bicyclo[3.2.2]nonan-4-carbonsäure-4-pyridin-2-yl-phenylester;
1,4-Diaza-bicyclo[3.2.2]nonan-4-carbonsäure-4-pyridin-3-yl-phenylester;
1,4-Diaza-bicyclo[3.2.2]nonan-4-carbonsäure-4-pyridin-4-yl-phenylester;
1,4-Diaza-bicyclo[3.2.2]nonan-4-carbonsäure-4-furan-3-yl-phenylester;
1,4-Diaza-bicyclo[3.2.2]nonan-4-carbonsäure-4-(6-fluor-pyridin-3-yl)-phenylester
1,4-Diaza-bicyclo[3.2.2]nonan-4-carbonsäure 4-imidazol-1-yl-phenylester;
1,4-Diaza-bicyclo[3.2.2]nonan-4-carbonsäure-4-[1,2,4]triazol-1-yl-phenylester;
1,4-Diaza-bicyclo[3.2.2]nonan-4-carbonsäure-2-benzooxazol-2-yl-phenylester;
1,4-Diaza-bicyclo[3.2.2]nonan-4-carbonsäure-2-benzothiazol-2-yl-phenylester;
1,4-Diaza-bicyclo[3.2.2]nonan-4-carbonsäure-4-(5-ethoxycarbonyl-pyridin-3-yl)-phenylester;
1,4-Diaza-bicyclo[3.2.2]nonan-4-carbonsäure-4-(6-methyl-pyridin-2-yl)-phenylester;
1,4-Diaza-bicyclo[3.2.2]nonan-4-carbonsäure-4-(3,5-dimethyl-isoxazol-4-yl)-phenylester;

1,4-Diaza-bicyclo[3.2.2]nonan-4-carbonsäure-4-(4-methyl-pyridin-2-yl)-phenylester;
1,4-Diaza-bicyclo[3.2.2]nonan-4-carbonsäure-4-(5-carbamoyl-pyridin-3-yl)-phenylester;
1,4-Diaza-bicyclo[3.2.2]nonan-4-carbonsäure-4-(5-cyano-pyridin-3-yl)-phenylester;
1,4-Diaza-bicyclo[3.2.2]nonan-4-carbonsäure-4-imidazo[1,2-a]pyridin-3-yl-phenylester;
1,4-Diaza-bicyclo[3.2.2]nonan-4-carbonsäure-3-pyridin-3-yl-phenylester.

**4.** Pharmazeutische Zusammensetzung, umfassend eine Verbindung nach Anspruch 1 und einen pharmazeutisch verträglichen Träger.

**5.** Verbindung nach Anspruch 1 zur Verwendung in einem Medikament.

**6.** Verwendung einer Verbindung nach Anspruch 1 bei der Herstellung eines Medikaments zur Behandlung von Schizophrenie bei einem Säuger.

**7.** Verwendung einer Verbindung nach Anspruch 1 bei der Herstellung eines Medikaments zur Behandlung einer Erkrankung oder eines Zustandes, ausgewählt aus entzündlicher Darmerkrankung, Colitis ulcerosa, Pyoderma gangrenosum, Morbus Crohn, Reizdarmsyndrom, spastischer Dystonie, chronischem Schmerz, akutem Schmerz, Zöliakie ("celiac sprue"), Pouchitis, Vasokonstriktion, Angst, Panikstörung, Depression, manisch-depressiver Krankheit ("bipolar disorder"), Autismus, Schlafstörungen, Jetlag, amyotropher Lateralsklerose (ALS), kognitiver Dysfunktion, Tinnitus, Hypertonie, Bulimie, Anorexie, Fettsucht bzw. Obesität, Herzrhythmusstörungen, Magensäurehypersekretion, Geschwüren, Phäochromocytom, progressiver supramuskulärer Paralyse ("progressive supramuscular palsy"), Chemikalienabhängigkeiten und - sucht, Abhängigkeiten von oder Sucht nach Nikotin (und/oder Tabakerzeugnissen), Alkohol, Benzodiazepinen, Barbituraten, Opioiden oder Kokain, Kopfschmerz, Schlaganfall, traumatischer Hirnverletzung (TBI), Psychose, Chorea Huntington, tardiver Dyskinesie, Hyperkinesie, Dyslexie, Multi-Infarkt-Demenz, altersabhängiger Abnahme der kognitiven Eigenschaften, Epilepsie, einschließlich Petit Mal-Epilepsie, HIVinduzierter Demenz, seniler Demenz vom Alzheimer-Typ (AD), Parkinsonkrankheit (PD), Aufmerksamkeits-Defizit-Hyperaktivität-Störung (ADHD bzw. ADHS) und Tourette-Syndrom bei einem Säuger.

**8.** Verbindung nach entweder Anspruch 1 oder Anspruch 2, worin Q wahlweise substituiert ist mit einem bis sechs Substituenten, unabhängig ausgewählt aus den Substituenten, die durch die Beispiele von Anspruch 3 veranschaulicht werden.

**Revendications**

**1.** Composé de formule

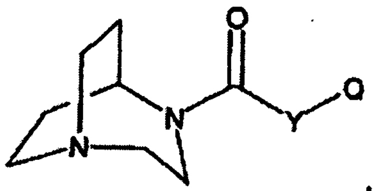

I

dans laquelle Y représente O ou un groupe NH ;

Q représente un groupe aryle en $C_6$ qui est substitué avec $R^3$ ;
$R^3$ représente un groupe hétéroaryle penta- à dodécagonal qui est facultativement substitué avec un à cinq substituants choisis indépendamment entre H, F, Cl, Br, I, nitro, cyano, $CF_3$, $-NR^6R^7$, $-NR^6C(=O)R^7$, $-NR^6C(=O)NR^7R^8$; $-NR^6S(=O)_2R^7$, $-NR^6S(=O)_2NR^7R^8$, $-OR^6$, $-OC(=O)R^6$, $-OC(=O)OR^8$, $-OC(=O)NR^6R^7$, $-OC(=O)SR^6$, $-C(=O)OR^6$, $-C(=O)R^6$, $-C(=O)NR^6R^7$, $-SR^6$, $-S(=O)R^6$, $S(=O)_2R^6$, $-S(=O)_2NR^6R^7$, alkyle en $C_1$ à $C_8$ à chaîne droite ou ramifié, alcényle en $C_2$ à $C_8$ à chaîne droite ou ramifié, alcynyle en $C_2$ à $C_8$ à chaîne droite ou ramifié, cycloalkyle en $C_3$ à $C_8$, cycloalcényle en $C_4$ à $C_8$, hétérocycloalkyle tri- à octogonal, bicycloalkyle en $C_5$ à $C_{11}$, bicycloalcényle en $C_7$ à $C_{11}$, hétérobicycloalkyle penta- à undécagonal, hétéro-bicycloalcényle penta- à undécagonal, aryle en $C_6$ à $C_{11}$, hétéroaryle penta- à dodécagonal et $R^6$ ;
chacun des groupes $R^6$, $R^7$ et $R^8$ est choisi indépendamment entre H, des groupes alkyle en $C_1$ à $C_8$ à chaîne

droite ou ramifié, alcényle en $C_2$ à $C_8$ à chaîne droite ou ramifié, alcynyle en $C_2$ à $C_8$ à chaîne droite ou ramifié, cycloalkyle en $C_3$ à $C_8$, cycloalcényle en $C_4$ à $C_8$, hétérocycloalkyle tri- à octogonal, bicycloalkyle en $C_5$ à $C_{11}$, bicycloalcényle en $C_7$ à $C_{11}$, hétérobicycloalkyle penta- à undécagonal, hétéro-bicycloalcényle penta- à undécagonal, aryle en $C_6$ à $C_{11}$ et hétéroaryle penta- à dodécagonal ; dans lesquels $R^6$, $R^7$ et $R^8$ sont chacun facultativement substitués indépendamment avec un à six substituants choisis indépendamment entre des substituants F, Cl, Br, I, nitro, cyano, $CF_3$, $-NR^9R^{10}$, $-NR^9C(=O)R^{10}$, $-NR^9C(=O)NR^{10}R^{11}$, $R^9S(=O)_2R^{10}$, $-NR^9S(=O)_2NR^{10}R^{11}$, $-OR^9$,- $OC(=O)R^9$, $-OC(=O)OR^9$, $-OC(=O)NR^9R^{10}$, $-OC(=O)SR^9$, $-C(=O)OR^9$, $-C(=O)R^9$, $-C(=O)NR^6R^7$, $-SR^6$, $-S(=O)R^6$, $-S(=O)_2R^6$, $-S(=O)_2NR^6R^7$, alkyle en $C_1$ à $C_8$ à chaîne droite ou ramifié, alcényle en $C_2$ à $C_8$ à chaîne droite ou ramifié, alcynyle en $C_2$ à $C_6$ à chaîne droite ou ramifié, cycloalkyle en $C_3$ à $C_8$, cycloalcényle en $C_4$ à $C_8$, hétérocycloalkyle tri- à octogonal, bicycloalkyle en $C_5$ à $C_{11}$, bicycloalcényle en $C_7$ à $C_{11}$, hétérobicycloalkyle penta- à undécagonal, hétérobicycloalcényle penta- à undécagonal, aryle en $C_6$ à $C_{11}$, hétéroaryle penta- à dodécagonal et $R^9$ ;

chacun des groupes $R^9$, $R^{10}$ et $R^{11}$ est choisi indépendamment entre H, des groupes alkyle en $C_1$ à $C_8$ à chaîne droite ou ramifié, alcényle en $C_2$ à $C_8$ à chaîne droite ou ramifié, alcynyle en $C_2$ à $C_8$ à chaîne droite ou ramifié, cycloalkyle en $C_3$ à $C_8$, cycloalcényle en $C_4$ à $C_8$, hétérocycloalkyle tri- à octogonal, bicycloalkyle en $C_5$ à $C_{11}$, bicycloalcényle en $C_7$ à $C_{11}$, hétérobicycloalkyle penta- à undécagonal, hétérobicycloalcényle penta- à undécagonal, aryle en $C_6$ à $C_{11}$ et hétéroaryle penta- à dodécagonal ;

ou un isomère, énantiomère, diastéréoisomère ou tautomère d'un tel composé, ou un sel pharmaceutiquement acceptable d'un tel composé ou isomère.

**2.** Composé suivant la revendication 1, dans lequel Y = O.

**3.** Composé suivant la revendication 1, qui est choisi parmi les composés suivants et leurs sels pharmaceutiquement acceptables :

ester 4-pyridine-2-yl-phénylique d'acide 1,4-diaza-bicyclo[3.2.2]nonane-4-carboxylique ;
ester 4-pyridine-3-yl-phénylique d'acide 1,4-diaza-bicyclo[3.2.2]nonane-4-carboxylique ;
ester 4-pyridine-4-yl-phénylique d'acide 1,4-diaza-bicyclo[3.2.2]nonane-4-carboxylique ;
ester 4-furanne-3-yl-phénylique d'acide 1,4-diaza-bicyclo-[3.2.2]nonane-4-carboxylique ;
ester 4-(6-fluoro-pyridine-3-yl)-phénylique d'acide 1,4-diaza-bicyclo[3.2.2]nonane-4-carboxylique ;
ester 4-imidazole-1-yl-phénylique d'acide 1,4-diaza-bicyclo[3.2.2]nonane-4-carboxylique ;
ester 4-[1,2,4]triazole-1-yl-phénylique d'acide 1,4-diaza-bicyclo[3.2.2]nonane-4-carboxylique ;
ester 2-benzoxazole-2-yl-phénylique d'acide 1,4-diaza-bicyclo[3.2.2]nonane-4-carboxylique ;
ester 2-benzothiazole-2-yl-phénylique d'acide 1,4-diaza-bicyclo[3.2.2]nonane-4-carboxylique ;
ester 4-(5-éthoxycarbonyl-pyridine-3-yl)-phénylique d'acide 1,4-diaza-bicyclo[3.2.2]nonane-4-carboxylique ;
ester 4-(6-méthyl-pyridine-2-yl)-phénylique d'acide 1,4-diaza-bicyclo[3.2.2]nonane-4-carboxylique ;
ester 4-(3,5-diméthyl-isoxazole-4-yl)-phénylique d'acide 1,4-diaza-bicyclo[3.2.2]nonane-4-carboxylique ;
ester 4-(4-méthyl-pyridine-2-yl)-phénylique d'acide 1,4-diaza-bicyclo[3.2.2]nonane-4-carboxylique ;
ester 4-(5-carbamoyl-pyridine-3-yl)-phénylique d'acide 1,4-diaza-bicyclo[3.2.2]nonane-4-carboxylique ;
ester 4-(5-cyano-pyridine-3-yl)-phénylique d'acide 1,4-diaza-bicyclo[3.2.2]nonane-4-carboxylique ;
ester 4-imidazo[1,2-a]pyridine-3-yl-phénylique d'acide 1,4-diaza-bicyclo[3.2.2]nonane-4-carboxylique ; et
ester 3-pyridine-3-yl-phénylique d'acide 1,4-diaza-bicyclo[3.2.2]nonane-4-carboxylique.

**4.** Composition pharmaceutique comprenant un composé suivant la revendication 1 et un support pharmaceutiquement acceptable.

**5.** Composé suivant la revendication 1, destiné à être utilisé en médecine.

**6.** Utilisation d'un composé suivant la revendication 1, dans la préparation d'un médicament destiné au traitement de la schizophrénie chez un mammifère.

**7.** Utilisation d'un composé suivant la revendication 1, dans la préparation d'un médicament destiné au traitement d'un trouble ou d'une affection choisi entre une maladie intestinale inflammatoire, la colite ulcérative, la pyodermie gangréneuse, la maladie de Crohn, le syndrome du côlon irritable, la dystonie spastique, la douleur chronique, la douleur aiguë, la sprue coeliaque, la diverticulite, la vasoconstriction, l'anxiété, le trouble panique, la dépression, un trouble bipolaire, l'autisme, des troubles du sommeil, le trouble dû au décalage horaire, la sclérose latérale amyotrophique (ALS), un dysfonctionnement cognitif, les bourdonnements d'oreilles, l'hypertension, la boulimie, l'anorexie, l'obésité,

les arythmies cardiaques, l'hypersécrétion d'acide gastrique, les ulcères, le phéochromocytome, la paralysie supramusculaire progressive, des dépendances ou accoutumances chimiques, des dépendances ou accoutumances à la nicotine (et/ou aux produits dérivés du tabac), à l'alcool, aux benzodiazépines, aux barbituriques, aux opioïdes ou à la cocaïne, les céphalées, un ictus, une lésion cérébrale traumatique (TBI), la psychose, la chorée de Huntington, la dyskinésie tardive, l'hyperkinésie, la dyslexie, la démence artériopathique, le déclin cognitif dû à l'âge, l'épilepsie, comprenant l'épilepsie sans petit mal, la démence induite par le VIH, la démence sénile de type Alzheimer (AD), la maladie de Parkinson (PD), le trouble d'hyperactivité avec déficit d'attention (ADHD) et le syndrome de Tourette chez un mammifère.

**8.** Composé suivant la revendication 1 ou 2, dans lequel Q est facultativement substitué avec un à six substituants choisis indépendamment parmi les substituants illustrés par les exemples de la revendication 3.